# EUROPEAN PATENT SPECIFICATION

(11) **EP 0 653 931 B1**
(45) Date of publication and mention of the grant of the patent: **22.09.1999**
(21) Application number: 93915554.5
(22) Date of filing: 27.07.1993
(51) Int. Cl.: A61K 7/09, A61K 47/42, A61K 47/36, C04B 33/18, C04B 33/28, C04B 33/34, C07G 17/00, C09D 199/00, C09J 199/00, C10M 159/02, A61K 7/48, C12P 19/04, C12N 5/04, E21B 43/25, E21B 33/138

(54) **INDUSTRIAL, PHARMACEUTICAL AND COSMETIC APPLICATIONS FOR CULTURED PLANT CELL GUMS**
INDUSTRIELLE, PHARMAZEUTISCHE UND KOSMETISCHE ANWENDUNGEN FÜR KULTIVIERTE PFLANZENZELLENGUMMEN
APPLICATIONS INDUSTRIELLES, PHARMACEUTIQUES ET COSMETIQUES DES GOMMES OBTENUES A PARTIR DE CELLULES DE PLANTES CULTIVEES

(30) Priority: 28.07.1992 US 920688
(43) Date of publication of application: 24.05.1995
(73) Proprietor: BIO POLYMERS PTY. LTD., Altona North, VIC 3025 (US); COMMONWEALTH SCIENTIFIC AND INDUSTRIAL RESEARCH ORGANISATION, Campbell, ACT 2612 (AU)
(72) Inventor: CLARKE, Adrienne, Elizabeth, Parkville, VIC 3052 (AU); BACIC, Antony, Eltham, VIC 3095 (AU); LANE, Alan, Gordon, Ashfield, NSW 2131 (AU)
(74) Representative: Woods, Geoffrey Corlett
(86) International application number: AU9300376
(87) International publication number: WO9402113

(56) References cited:
- EP-A- 0 062 457
- EP-A- 0 079 794
- AU-A- 1 021 492
- AU-A- 1 394 888
- GB-A- 749 766
- US-A- 1 873 631
- US-A- 2 093 405
- US-A- 2 803 558
- US-A- 2 884 335
- PATENT ABSTRACTS OF JAPAN vol. 16 no. 437 (C-0984) ,11 September 1992 & JP-A-04 152890 (MITSUI ENG & SHIPBUILDING CO) 26 May 1992,
- PATENT ABSTRACTS OF JAPAN vol. 7 no. 155 (C-175) ,7 July 1958 & JP-A-58 067190 (LOTTE) 21 April 1983,
- BACIC, A. et al., Phytochemistry, Vol. 27, No. 3, issued 1988, (Pergamon Journals Ltd), "Arabinogalactan Proteins from Stigmas of Nicotiana Alata", see pages 679-684.
- GLEESON, P.A. et al., Biochemical Journal, Vol. 264, issued 1989, (Portland Press Ltd, USA), "Characterization of the Hydroxyproline-Rich Protein Core of an Arabinogalactan-Protein Secreted from Suspension-Cultured Lolium Multiflorum (Italian Ryegrass) Endosperm Cells", see pages 857-862.
- PATENT ABSTRACTS OF JAPAN, C-403, page 5; & JP,A,61 209 599 (NITTO ELECTRIC IND CO LTD) 17 September 1986 (17.09.86).
- PATENT ABSTRACTS OF JAPAN, C-477, page 46; & JP,A,62 201 594 (NITTO ELECTRIC IND CO LTD) 5 September 1987 (05.09.87).
- DERWENT ABSTRACT Accession No. 93-131307/16, Class D17; & JP,A,05 070 503 (KAO CORP) 2 March 1993 (02.03.93).
- PATENT ABSTRACTS OF JAPAN, C-965, page 84; & JP,A,04 099 742 (HONSHIYUU KAGAKU KOUGIYOU K.K.) 31 March 1992 (31.03.92).
- PATENT ABSTRACTS OF JAPAN, C-135, page 61; & JP,A,57 133 170 (KIKUSUI KAGAKU KOGYO K.K.) 17 August 1982 (17.08.82).
- ANDERSON, D.M.W. et al., Carbohydrate Research, Vol. 11, issued 1969 (Elsevier Publishing Company, Amsterdam), "An Analytical Study of Gum Exudates from the Genus Araucaria Jussieu (Gymnospermae)", see pages 43-51.

## Description

### Field of the Invention

The subject invention relates to the use of cultured plant cell gums in applications in oil and gas well drilling and production, and in the pharmaceutical, textile, printing ink, lithography, cosmetic, adhesive, paper, paint, ceramic and cleaning detergent industries.

### Background of the Invention

A variety of natural and semisynthetic complex carbohydrates or polysaccharides have been commercially important in human and pet food manufacturing; in the cosmetic, paper, textile, paint, agricultural, explosives, hydrolube, adhesive, ceramic, cleaning polish, detergent, fire fighting, ink, photography, lithography, and deodorant gel industries; and in mining, and gas well drilling and production. Natural complex carbohydrates and polysaccharides include seaweed extracts, plant exudates, seed or root extracts, and microbial polysaccharides produced by fermentation. Semisynthetic complex carbohydrates and polysaccharides include cellulose derivatives, low-methoxyl pectin, propylene glycol alginate, triethanolamine alginate and guar gum derivatives. Sandford, P. & Baird, J. (1983) "Industrial Utilization of Polysaccharides" in The Polysaccharides, Vol. 2, pp. 411-491.

The production of natural complex carbohydrates or polysaccharides is frequently problematic. For plant exudates, seed or root extracts, production is dependent on climate and harvest conditions. For example, gum arabic is an exudate from Acacia senegal trees. Gum production is stimulated by stripping the bark from the trees; the gum is collected by hand in the form of "dried tears." Production of gum arabic can vary each year as a function of weather conditions, labor strikes, natural disasters, etc. Meer et al. (1975) Food Technology 29:22-30. The unreliable supply results in variable gum arabic cost. Seed gums, such as guar gums are expensive due to harvesting costs. Guar gum is derived from the seed of the guar plant Cyamopsis tetragonolobus. Processing involves removal of the seed coat, separation of the germ from the endosperm, and milling of the endosperm. Sandford, P. & Baird, J. (1983), supra.

The production of seaweed extracts can also be problematic. Agar production is labor intensive in that it involves the harvesting of red seaweed by hand: in some areas of the world, divers in full pressure suits collect individual plants in deep water; in other places, the seaweed can be collected at low tide without the use of diving equipment. Carrageenan or Irish Moss is produced from another red seaweed harvested by raking and hand gathering. Algin is produced from brown algae which can be harvested manually or with small mechanical harvesters. Sandford, P. & Baird, J. (1983), supra.

Further, hand harvesting can introduce a purity problem. For example, hand collected lots of gum arabic are seldom pure; samples are classified according to grade which depends on color, and contamination with foreign bodies such as wood or bark (VanNostrand's Scientific Encyclopedia, 7th ed. (1989) D. Considine (ed.), Vol. I, p. 1389).

Microbial fermentation gums such as xanthan gum avoid many of the difficulties associated with harvesting of plant exudates or extraction of algae because production is carried out in fermentation facilities. However, xanthan gum production poses other problems. Xanthan gum is produced by Xanthamonas campestris, which presents a cell disposal problem because X. campestris is a plant pathogen (Scaad, N.W. (1982) Plant Disease 66(10):882-890). Xanthan gum has also been objected to as being too expensive for certain applications such as drilling mud. See, e.g., Kirk-Othmer Chemical Engineering Encyclopedia (3rd. ed. 1981) 17:153.

Thus, there is a clear need in a number of industries for a reliable, relatively inexpensive gum or class of gums that do not create a disposal problem. While a number of plant cells have been observed to produce polysaccharide and/or complex carbohydrates when cultured (Aspinall, G. & Molloy, J. (1969) Canadian J. Biochem. 47:1063-1070; Fincher, G. et al. (1983) Ann. Rev. Plant Physiol. 34:47-70; Clarke, A. et al. (1979) 18:521-540; McNeil, M. et al. (1984) Ann. Rev. Biochem. 53:625-663; Hale, A. et al. (1987) Plant Cell Reports 6:435-438; and Bacic, A. et al. (1987) Australian J. Plant Physiol. 14:633-641), it has not been suggested that such cultured plant cell gums might be suitable in the pharmaceutical, paper, textile, paint, agricultural, explosives, hydrolube, adhesive, ceramic, cleaning polish, detergent, fire fighting, ink, photography and lithography industries; or in mining, and oil and gas well drilling and production. Only Otsuji, K. et al. EP 0 285 829 (published October 12, 1988) have utilized cultured Polianthes gum in cosmetic applications.

### Summary of the Invention

The subject invention comprises the use of cultured plant cell gums of vascular plants in a variety of industrial, pharmaceutical and cosmetic applications including, without limitation, textiles, paper, adhesives, inks, lithography, ceramics, cleaning detergents, firefighting, agricultural, explosives, oil and gas wells, and cosmetics. Any cultured plant cell gum of a vascular plant can be useful in the subject industrial, pharmaceutical and cosmetic applications. Plant cell lines that produce at least about 0.05% (w/v) gum in the final fermenter culture broth, are preferred to reduce production costs. Plant cell lines that produce at least about 0.5%, 2.0%, and 10.0% (w/v) gum in the final culture broth are increasingly preferred. In one embodiment, the cultured plant cell gums employed in such applications are cultured plant cell gums having arabinogalactan proteins (AGPs) of at least about 4.0% (w/w). In other embodiments, cultured plant cell gums of Phleum, Nicotiana, Pyrus, and Lolium, are employed as viscosifiers, as thickening, gelling, emulsifying, dispersing, suspending, stabilizing, encapsulating, flocculating, film forming, sizing, adhesive, binding and/or coating agents, and/or as lubricants, water retention agents and coagulants. As discussed herein, culture conditions for the plant cells can affect functional properties of the gum product.

Cultured plant cell gum products can be used as a substitute for prior art guns, such as gum arabic and guar gum. The cultured plant cell gums can also be used as a substitute for xanthan gum, alginic acid, agar, calcium alginate, carrageenan, guar gum, karaya gum, locust bean gum, potassium or sodium alginate, tragacanth gum and others. For example, the cultured plant cell gums can be used as thickening agents and/or emulsifying agents to replace gum arabic in adhesives, inks, textile printing and cosmetics. The cultured plant cell gums can be used to replace alginic acid as an emulsifier, thickening agent, suspending agent, waterproofing agent, etc. in toothpaste, cosmetics, pharmaceuticals, textile sizing, coatings, oil-well drilling muds, and concrete. The cultured plant cell gums can be used to replace agar as a gelling agent, protective colloid, in photographic emulsions or other applications. The cultured plant cell gums can be used to replace calcium alginate as a thickening agent, stabilizer, etc. in synthetic fibers. Carrageenan, which can be used as an emulsifier, protective colloid, stabilizing agent, etc. in toothpastes, cosmetics and pharmaceuticals, can be replaced by cultured plant cell guns. Cultured plant cell gums can substitute for guar gum, which functions as a thickening agent, emulsifier, etc. in paper, cosmetics, pharmaceuticals, textiles, printing, polishing, and as a fracture aid in oil wells. Cultured plant cell gums can also replace karaya gum as a protective colloid, stabilizer, thickener, emulsifier, etc. in pharmaceuticals, textile coatings and adhesives. Cultured plant cell gums can replace locust bean gum (carob-bean gum) as a stabilizer, thickener, emulsifier, etc. in packaging material, cosmetics, sizing and finishes for textiles, pharmaceuticals and paints. Potassium or sodium alginate, which can function as an emulsifier, thickening agent, stabilizer, etc. in pharmaceuticals, textile printing, cement compositions, paper coatings, and in some water-base paints, can be replaced by cultured plant cell guns. Cultured plant cell gums can replace tragacanth gum as on emulsifying agent, coating agent, thickening agent, stabilizer, etc. in pharmaceuticals, adhesives, leather dressings, textile printing and sizing, dyes, toothpastes, hairwave preparations, soap chips and powders. Xanthan gum, which is used as a thickening, suspending, emulsifying agent, stabilizing agent, etc. in oil and gas well drilling muds and other applications, can also be replaced by cultured plant cell gums. In replacing such prior art gums, the cultured plant cell gums can offer unexpectedly improved results. Often, cultured plant cell gums can surprisingly be used in smaller quantities than the prior art gums to achieve equivalent functional results. Further, production of the cultured plant cell gums do not present the cell disposal problem that xanthan gum production does.

The cultured plant cell gums are not useful in applications where their utilities or properties are significantly compromised or destroyed. Organic solvents such as alcohol, acetone and ether and the like can disrupt function by causing precipitation of the cultured plant cell gums. To maintain the gums' emulsification, thickening or gelling properties, it is preferred that the temperature of the gum-containing solution or mixture be maintained between about 4° and 90°C and have a pH of neutral to slightly alkaline. As the pH increases, the thickening capacity of the gums decreases. However, even at elevated pH, viscosity can increase with increased ionic strength. Gum-containing solutions can gel in the presence of divalent cations such as calcium, and as temperature decreases, gel strength increases. Typically, stable gels are produced in the pH range of between about 3 to 10 and in the presence of calcium ions. Further, heating and cooling of gelled gum solutions between ambient and 80°C has not reduced gel strength, indicating that the gels can be thermoreversible.

In general, the cultured plant cell gums are useful in a wide variety of applications because they are stable over a wide range of temperatures. In an emulsion or solution, the gums are functional over a temperature range of about 0° to 100°C at neutral pH. The dried gum powder (neutral pH) is stable over a temperature range of about -70°C to about 10°C. If heated, the dried, powdered gum can caramelize.

### Brief Description of the Figures

**Figure 1** is a plot of viscosity as a function of shear rate for *N*. *plumbaginfolia* gum NP5-1000 at concentrations: 0.1% w/w (○), 0.25% w/w (□), 0.5% w/w (△), 1% w/w (◇), and 2% w/w (∇).
**Figure 2** is a plot of viscosity as a function of shear rate for *P*. *communis* gum P7-1000 at concentrations: 0.1% w/w (○), 0.25% w/w (□), 0.5% w/w (△), 1% w/w (◇), and 2% w/w (∇).
**Figure 3** is a plot of viscosity as a function of shear rate for Timothy grass gum at concentrations: 0.5% w/w (○) and 1% w/w (□).
**Figure 4** is a plot of viscosity as a function of shear rate for *N*. *plumbaginifolia* gum NP5-1000 (1% w/w) for pH values of 2.32 (○), 2.86 (●), 4.77 (∇), 7.09 ( 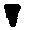 ) and 11.02 (□).
**Figure 5** is a plot of viscosity as a function of shear rate for *P*. *communis* gum P7-1000 (1% w/w) for pH values of 2.32 (○), 3.0 (●), 4.36 (∇), 7.01 ( 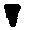 ) and 11.28 (■).
**Figure 6** is a plot of viscosity as a function of shear rate for Timothy grass gum (1% w/w) for pH values of 2.88 (○), 4.93 (■), 6.95 (△) and 9.06 (◇).
**Figure 7** is a plot of viscosity as a function of shear rate for *N*. *plumbaginifolia* gum NP5-1000 (1% w/w) for temperature of 10°C (○), 20°C (●), 40°C (∇), and 60°C ( 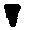 ).
**Figures 8** to **10** are plots of viscosity as a function of shear rate for *N*. *plumbaginifolia* gum product NP5-1000 showing effect of addition of 1%, 2% and 10% NaCl at 1% w/w gum concentration (Fig.8); effect of addition of 10% NaCl at 0.1%, 0.5% and 1.0% (w/w) gum concentrations (Figs. 9 and 10).

### Detailed Description of the Invention

Prior International Patent Application No. PCT/AU88/00052, dated February 26, 1998, disclosed the general ability of cultured plant cell gums to function as emulsifying agents, thickeners, stabilizers, texture modifiers, gelling agents, binding or coating agents, and suspending agents in various food product applications. The present work specifically describes and exemplifies non-food industrial, pharmaceutical and cosmetic applications of cultured plant cell gums.

"Cultured plant cell gum" is defined as the substantially cell-free material recovered from cultured plant cells of vascular plants, and is used interchangeably herein with "gum product." The cultured plant cells are those which are capable of synthesizing components of the gum product and transporting the same extracellularly in culture. A variety of vascular plant cells, including those derived from gymnosperms and angiosperms, may be used in the subject method. Cells of plants of the Dicotyledonae class (e.g., the Rosidae and Asteridae subclasses) and Monocotyledonae class (e.g., the Commelinidae subclass) an be used in the subject methods. Pyrus, Prunus, Rosa, Nicotiana and Phleum cell lines can produce gums having the preferred concentrations of polysaccharide and/or AGPs. In particular, Pyrus communis, Prunus avium, Rosa glauca, Nicotiana plumbaginofolia, Nicotiana alata Phleum pratense cell lines can produce gums that can be useful in the subject methods.

The cultured plant cell gum comprises complex carbohydrates and optionally glycoproteins, which are secreted into the medium by the cultured cells. The major classes of complex carbohydrate polymers are proteoglycans (e.g., arabinogalactan proteins (AGPs)), polysaccharides (e.g., neutral and acidic pectins), hetero- and homo-glucans, heteroxylans, and hetero- and homo-mannans (McNeil et al. (1984) Ann. Rev. Biochem. 53:625-633). Complex carbohydrates and glycoproteins are known to be secreted by many cultured cell lines (Clarke, A. et al. (1979) Phytochemistry 18:521-540; Fincher et al. (1983) Ann. Rev. Plant Physiol. 34:47-70; Bacic, A. et al. (1987) Australian J. Plant Physiol. 14:633-641).

The cells to be cultured can be initiated from, for example, a leaf, style, anther or stem of a plant, segments of which can be placed on solid culture. Callus cells may proliferate from any of the tissues of these organs and the callus cells can then be transferred to liquid suspension culture. Alternatively, seeds an be surface sterilized, and placed in a solid or liquid culture to initiate germination. The germinating seedlings can then be maintained, for a time, in liquid suspension culture. The suspension culture medium can be any known suitable medium such as MS medium (Mirashige, T. & Skoog, F. (1962) Physiologia Plantarum 15:473-497; Wu, M. & Wallner, S. (1983) Plant Physiol. 72:817-820). Transfer to suspension culture is preferred because in general it increases gum production and because it is possible to scale up a liquid suspension culture. Air fermenters are preferred because they reduce shear stress on the cells. While cells can produce gum on a solid medium, mass culture on solid media poses a number of practical difficulties, including gum collection. Usually, a plant cell hormone is employed to enhance cell growth and/or polysaccharide production. Plant hormones include, for example, the auxins such as 2,4-dichlorophenoxyacetic acid (2,4-D) and 2,4-dichlorophenoxybutyric acid (2,4-DB). The specific culture conditions for N. plumbaginifolia, P. communis and P. pratense are exemplified herein.

It has been observed employing BLM as a carbon source increases cell growth and gum yield. It has also been observed that an increase in osmotic pressure or in sucrose concentration in the medium can increase gum production by some cultured plant cells.

The gum product can be recovered from the culture medium by methods will known in the art. See Johns, M. & Noor, E. (1991) Aust. J. Biotechnol. 5(2):73-77; Golueke, C. et al. (1965) U.S. Patent No. 3,195,271; Saviour, R. & Kristiansen, B. (1983) Eur. J. Appl. Microbiol. Biotechnol. 17:178-181; Mort, A. et al. (1991) Carbohydrate Res. 215:219-237; and Wu, M. & Wallner, S. (1983) Plant Physiol. 72:817-820. A specific recovery and purification method is exemplified herein. A "complexant" is a composition or compound that sequesters calcium or other divalent metal ions from the gum product during the recovery procedure. For example, Na₂·EDTA added during the recovery process chelates calcium. Other sequestering agents such as citrate, cyclohexane diamine tetraacetate (CDTA), imidazole, sodium hexametaphosphate may also be used. Sequestering of calcium is desirable to avoid the formation of insoluble complexes during drying of the recovered gum.

The skilled practitioner, using information available in the art and the teachings of the subject application, can identify cultured plant cell gums that are useful as thickening, galling, emulsifying, dispersing, suspending, stabilizing, encapsulating, flocculating, film forming, sizing, adhesive, binding and coating agents, and as lubricants, water retention agents and coagulants, etc. in the aforementioned industries. The suitability of using a cultured plant cell gum for a particular application can be assessed by methods known to those of skill in the art.

The cultured plant cell gums can be used to establish and stabilize solid, liquid and gaseous dispersions. An emulsion is an intimate mixture of two immiscible liquids in which one phase is dispersed throughout the other as small, discrete droplets (Sandford, P. & Baird, J., "Industrial Utilization of Polysaccharides" in The Polysaccharides (1983), Academic Press, Inc., Vol 2, pp. 411-491). The cultured plant cell gums can be used as emulsifying agents or stabilizing agents in emulsions. Suspensions are solid particles dispersed uniformly throughout a liquid phase (a suspension) mainly by increasing the viscosity of the suspension liquid phase with suspending agent. Foams are gas dispersed in a liquid or solid phase. When cultured plant cell gums are employed as foam stabilizers, they affect the surface properties (e.g., interfacial tension) of foams, thereby promoting a firm, stable foam.

Emulsification capacity can be assessed by, for example, measuring the reduction in aqueous surface tension or interfacial tension due to the gum product, measuring the critical micellar concentration (CMC), or measuring the hydrophile-lipophile balance (HLB; the ratio of polar to nonpolar portions of the composition). Additional methods of assessing emulsifying capacity include particle sizing and counting, and affect on viscosity and electrical properties of the emulsion due to the gum product. For a discussion of such methods, sea Zajic, J. & Panchal, C. in CRC Critical Review in Microbiology (1976), pp. 39-66. The choice of a particular gum product for a desired application depends on additional factors such as solubility and compatibility with other chemicals in the emulsion mixture, and pH, ionic strength and temperature of the emulsion mixture. The specific method employed to measure the emulsification capacity for at least some of the gum products described herein involves measurement of turbidity and droplet size and is described in the Examples.

Emulsion stabilizing capacity is the ability of a gum to maintain an emulsion over time. Emulsion stability can be tasted by evaluating the turbidity of the emulsion (or industrial emulsion mixture) over time.

Thickening agents increase the viscosity of aqueous solutions or suspensions. They increase the resistance to flow of a liquid. Sandford, P.A. & Baird, J., supra. Viscosity imparted by cultured plant cell gums to mixtures or solutions can be measured with commercially available viscometers. Such viscometers commonly employ methods based on Stoke's law, the capillary tube method, the rotating cylinder method or the oscillating disk method. The specific method employed to measure the viscosity of at least some of the gum products described herein is described in the Examples.

Assessment of galling capacity of a gum product can be carried out by methods known in the art. The specific method employed to measure gelling capacity of at least some of the gum products described herein is set forth in the Examples. Gelling capacity can be assessed by measuring the rupture strength, shear modulus, back extrusion and melting and setting points of the gum product.

Lubricating capacity can be assessed by methods known in the art. For example, an adaptation of ASTM (American Society for Testing Materials) Method D4172 may be used.

Encapsulating capacity can be assessed by methods known in the art. For example, the gum may be tested as an encapsulating agent for a flavor oil in a powdered drink mix, and compared to an encapsulating agent commonly employed in spray-dried flavor oil preparations, such as the starch-based encapsulating agent, N-Lok, for example as described in Example 3.D. hereof.

In some cases, particular functional properties have been associated with particular gum components. It has been observed that AGP in the cultured plant cell gum product can enhance emulsification properties. For example, Pyrus communis and Nicotiana plumbaginifolia have higher levels (6-11 % (w/w)) AGPs, while Phleum pratense produces a gum with nondetectable AGP and poor gelling and emulsification capacity. Phleum pratense has comparable viscosity to Pyrus and Nicotiana gums without the gelling and emulsification properties. Phleum pratense is thus useful as a viscosity enhancer in applications where emulsification is not desired, e.g., in applications where guar gum and hydroxymethylcellulose have traditionally been used.

Those embodiments of the subject invention which use the gum products as emulsifiers preferably employ a gum product relatively rich in AGPs. In particular, cultured plant cell gums containing at least about 4% (w/w) AGP in the gum can be useful. Complex carbohydrates in the culture fluid can be determined by the method of Dubois et al. (1956) Anal. Chem. 28:350-356. AGP can be determined by the method of Van Holst, G. & Clarke, A. (1985) Anal. Biochem. 148:446-450. AGP-containing gums have been found in higher plants (14 orders of angiosperms, 3 orders of gymnosperms), and in lower plants (e.g., Fontinalis anti-pyretica). Fincher, G. et al., supra.

It has been found that a gum product recovered from Pyrus communis cells suspension cultured in MS medium plus 2,4-D has complex carbohydrates at about 5.26 mg/ml of culture fluid as determined by the method of Dubois et al. (1956) Anal. Chem. 28:350-356; and 8.9% (w/w) AGP as determined by the method of Van Holst et al. (1985) Anal. Biochem. 148:446-450.

The cultured (MS medium) gum product Lolium multiflorum and Nicotiana plumbaginifolia have been found to have an AGP % (w/w) of 11.0 and 4.5, respectively. In contrast, cultured cells (MS medium) of Phleum pratense have been found to have no detectable AGP (detection limit is about 0.25 µg by the method of Van Holst et al. (1985)).

A description of particular applications in which the cultured plant cell gums can be employed follows. This discussion is not intended to be limiting.

In the paper industry, prior art gums have been used in wet end beater aids, surface sizes (e.g., size press and calender), pigmented coatings (e.g., blade, roll airknife, and size press coatings), and in adhesives. Sandford, P. & Baird, J., supra. Cultured plant cell gums can be used as substitutes for such prior art gums as locust bean gum, karaya and guar gums as hydrophilic colloids employed in the wet end as beater aids to reduce flocculation of pulp suspensions and improve paper formation. The cultured plant cell gums can also replace prior art gums as a surface size which is typically applied after the formation of the sheet at calender rolls or at the size press. Sandford, P. & Baird, J., supra. As surface sizes, cultured plant cell gums can impart water resistance, oil and solvent resistance, glue holdout, scuff resistance, physical strength curl control and gloss. The cultured plant cell gums can also replace such prior art polysaccharides as sodium alginate, which is used as a thickener and dispersant in the pigment coating. The purpose of such an additive is to prevent agglomeration, and to produce adequate flow and leveling of the coating, and to prevent pattern or orange peel in the coating. Sandford, P. & Baird, J., supra.

As exemplified herein, addition off the cultured plant cell gums as a beater aid at the wet end has been observed to result in superior tensile and burst strength, improved resistance to erasure, reduced lint on the paper surface and reduced rate of water penetration as compared paper manufactured without a beater aid. Without wishing to be bound by theory, it is believed that at least some of these improvements are due to a more uniform distribution of pulp fines.

In the adhesives industry, some prior art gums, waxes, tars, and natural resins have functioned as adhesives when dissolved or dispersed in water or organic solvents, applied between substrates and the solution/dispersal allowed to undergo solvent evaporation. Cultured plant cell gums have been found suitable for use in a water re-moistenable adhesive for paper or aluminum foil sheets. The cultured plant cell gum increases viscosity, thereby moderating the flow during application, and the finished film thickness and water retention. The gum product may also serve as a surface attaching agent. The cultured plant cell gum adhesive, when dried on the surface of paper or aluminum sheets, has good affinity for water and does not cause discoloration of the paper or become brittle on aging. The concentration range in the liquid adhesive concentration is between about 1.0 and 3.0% (w/v). The cultured plant cell gums can be used as an adhesive or cement in other applications.

Prior art gums have also been employed in oil and gas field applications including drilling, well completion (cementing and stimulation) and enhanced oil recovery. As used herein, "oil and gas well fluids" refers to all oil and gas well development or production fluids, including without limitation drilling fluids, cementing fluids, and enhanced oil recovery injection fluids. Drilling fluids or muds function to transport drill cuttings to the surface, control formation pressures, maintain bore hole stability, protect productive formations and cool and lubricate the bit and drill string. Prior art gums have been used to impart greater viscosity to the drilling fluid, to act as suspending agents for cuttings and weighting materials, and to reduce loss of water or fluid by preventing penetration into the rock formation. The rheological requirements off the drilling fluid are that it have low viscosity at high shear rates (i.e., at the drill bit), but high pseudoplasticity to suspend solids in laminar flow. When mud circulation stops, the gel strength is preferably sufficient to suspend solids. Sandford, P. & Baird, J., supra; and Kirk-Othmer Chemical Engineering Encyclopedia (3rd. ed. 1981) 17:143-166. These rheology requirements have previously been addressed with combinations of bentonite, cellulose ethers, polyacrylamides and xanthan gum. Drilling mud additives for reduction of fluid loss have included carboxymethylcellulose, polyacrylates and xanthan gum. During well cementing, a cement lining is installed to isolate the productive zone from the remainder of formations. Fluid loss additives are also used during this stage to prevent cement dehydration and minimize water loss to the formation. Sandford, P. & Baird, J., supra. Following drilling and cementing, a completion may be used to remove undesirable formation particles and debris and prevent permeability damage to the producing zone. Completion fluids contain salts for density, and viscosifiers such as xanthan gum to provide suspension for the removal of debris. During well stimulation, hydraulic fracturing and/or acidizing fluids can be used to enhance hydrocarbon productivity. Hydraulic fracturing fluids require suspending agents such as guar or xanthan gums to carry propping solids. Acidizing fluids require a gelling agent effective in high acid concentrations (e.g., 15% HCl). In enhanced oil recovery, the injection fluids contain polymers to increase viscosity, resulting in better oil displacement. Xanthan gum has been a common component in enhanced oil recovery polymer flooding.

It has now been found that cultured plant cell gums can be employed in drilling fluids to increase viscosity, and as emulsifying, suspending, lubricating agents and fluid loss reduction agents. As an emulsifying agent in a drilling mud, the cultured plant cell gums can emulsify and stabilize oil-in-water or water-in-oil mixtures. As a suspending agent, the cultured plant cell gums disperse and suspend cuttings and weighting materials so as to provide a protective colloid for well equipment. As a lubricating agent, cultured plant cell gums can reduce frictional resistance between the drill string and the formation or casing or during string raising and lowering. The strong water affinity of the gum products can prevent water filtration into surrounding strata during drilling or cementing phases. The gum products can also be used as viscosifiers in completion fluids. In hydraulic fracturing fluids, the cultured plant cell gums can be used to impart viscosity, suspend propping solids and as gelling agents. In enhanced oil recovery, cultured plant cell gums can be used to increase viscosity of the injection fluid. The concentration of cultured plant cell gum in the drilling mud, completion, fracturing and enhanced oil recovery injection fluid is between about 0.1 and 3.0% (w/v). For P. communis gum, a soft gel begins to form at about 0.5% (w/v).

For each of the foregoing oil drilling applications, the whole fermentation mixture may be used, i.e., without removal of cells. This alternative has the advantage of simplifying the manufacture of oil and gas well fluids. The biodegradability and non-pathogenic nature of the cells makes such alternative possible.

An additional advantage of using cultured plant cell gums in oil and gas field fluids is that they have much less environmental impact than those using palm oil. This is particularly the case for drilling muds prepared for off-shore drilling where it is desirable that leakages from the well be easily dissipated. Aqueous-based drilling muds dissipate more effectively than oil- based muds.

In ink formulations, thickening, suspending and/or emulsifying agents are used to provide the proper viscosity for application and to increase the stability of the ink. Lithographic, letterpress and screen printing inks have higher viscosities and frequently contain thickeners. Flexographic (flexo) and rotogravure (gravure) printing inks have lower viscosities, but use emulsifying or suspending agents for uniform distribution of the pigment and to prevent the ink from separating. Flexographic inks can be alcohol or water based emulsions. Rotogravure inks also contain an emulsion and have the advantages of excellent press stability, printing qualities, the absence of fire hazard and the convenience and economy of water for reduction and cleanup. The ink distribution systems of flexo and gravure printing presses are simple and do not provide the means to distribute and level highly viscous inks; therefore, viscosity is typically 5-100 cP. Letterpress and litho inks can vary in viscosity from under 500cP for a letterpress-type news ink to over 500 P for special litho ink formulations. In lithography and letter press, uniform and adequate transfer of ink to the printing plate is ensured by a multitude of rollers in the ink distribution unit. Rheology of the litho and letterpress inks is therefore important to roller-to-plate transfer, fidelity in printing, drying speed, holdout, and trapping properties obtained on the substrate. In general, higher press speeds require lower viscosity inks and slower press speeds employ more viscous inks. Low viscosity ink is used in fine-line flexography and shallow-cell gravure printing. Printing smooth, dense solids can best be achieved using higher viscosity ink. Rheology is also important as a color strength determinant. Over-pigmentation leads to a more thixotropic ink, thereby creating a balancing relationship between color intensity and rheology. Kirk-Othmer Chemical Engineering Encyclopedia, supra, Vol. 13, pp.374-376. Lasday, S. (ed.) Handbook for Graphic Communications: (1972) Ink, Paper, Binding, Vol 6., pp. 6-13.

It has been found that cultured plant cell gums can be used as emulsifying, suspending and/or thickening agents in a variety of printing inks, including litho, letterpress, screen printing, flexographic and gravure inks. The gum concentration in flexo inks can be between about 0.5 and 4.0% (w/v).

Additionally, in offset lithography, prior art gums have been used as emulsifying agents and viscosifiers in lithography solutions. Offset lithography is a planographic process where the image and non-image are in the same plane. The image area is oil receptive and the non-image area is water receptive so that following wetting of the plate with the fountain solution, the ink, when rolled across the plate will only be attracted to the oil receptive areas. As used herein, "lithography solution" refers to any non-ink solution used in lithography, including fountain solutions, sensitizing solutions and protecting solutions. The fountain solution is a desensitizing solution which prevents ink from adhering to the plate. Fountain solutions have contained gum arabic, typically at 0.2% (w/w). Lasday, S., supra, Vol. 6, pp. 93-95. The desensitizing use of gum arabic has taken advantage of the good wettability imparted to the fountain solution and also of the viscosity control that allows the wash solution to cling to the plate without running off or forming isolated droplets or pools on the plate. On metal plates, the desensitizing effect might be caused by the formation of an insoluble film of EG Aluminum or Zinc Arabate. A more plausible explanation is that the film of gum is absorbed by the plate. Studies have shown that such films occur on plates of zinc, aluminum, copper, silver, iron tin, lead, glass and fused silica. These films are not mono-molecular but are composed of many molecular layers. LSC Printing Inks, Reinhold Publishing Corporation, New York (1940) pp. 230, 334, 346, 398-9 and 417. Measurement of the wettability of the desensitizing solutions can be evaluated by measurement and study off the contact angles. In this process a section of the plate is partially immersed in water or in a solution of the gum to be tested. The plate is then turned at an angle to the surface of the liquid until the meniscus appears to be eliminated. The resulting angle of the plate to the surface of the liquid is known as the contact angle and is the measure of the wettability of that particular plate with the solution being tested. Read REF Modern Lithography (1951) 47:62.

Cultured plant cell gums can be used as emulsifying agents in sensitizing or fountain solutions for the plates during operation and in protecting solutions during storage. The concentration range of the gum in fountain solutions is between about 0.01 and 2.0% (w/w).

In the textile industry, gums have been used as sizing and thickening agents. Sizing agents act during textile manufacture by binding the loose fibers of the warp, thereby imparting strength, flexibility and smoothness to the warp, allowing weaving to proceed efficiently. Thickeners control the viscosity of various formulations used in the textile industry including dyes, printing inks, coating and flocking solutions. Prior art gums, including guar, algin and xanthan gums have been used in printing and dyeing solutions. Sandford, P. & Baird, J., supra. Cultured plant cell gums can be useful as sizing or thickening agents in the textile industry. As exemplified herein, the gum product can function as a thickening agent for dyestuff used in wool and cotton fabric printing. The concentration range of the gum product in the dyestuff is between about 0.1 and 5.0% (w/v). Modified approaches can be used in the reactive dyestuff process and direct vat dyestuffs for silk and hydrophobic man-made fibers (nylon, acrylics, polyester and acetates).

In the paint industry, viscosifiers, thickeners, emulsifying agents, suspending agents, and dispersants are used to improve flow properties of the paint so that a smooth coat of desired thickness can be applied to a vertical surface without sagging, and to stabilize the paint by preventing coagulation and pigment settling. Thixotropic character of the paint is important in providing good levelling, prevention of running, and avoidance of segregation or stratification of the paint during storage. Sandford, P. & Baird, J., supra; and Gamble, D. & Grady, D., U.S. Patent No. 2,135,936 (1938). As exemplified herein, cultured plant cell gums can be used as emulsifying agents in on acrylic resin paint or an oil emulsion paint. The concentration range of the gum product in acrylic or oil based paint is between about 0.2 and 0.3% (w/v).

In ceramics manufacturing, a glaze or a colored, opaque or transparent coating is applied to the ceramics before firing. The glaze forms a hard, nonporous surface. Glazes are usually made from powdered glass combined with colored oxides of such elements as cobalt, chrome, manganese or nickel. The mixture of powders is suspended in water and applied to the ceramic surface by spraying brushing or dipping. The glaze is then dried and fixed onto the ceramic surface by firing. Emulsifying agents, suspending agents or dispersants can be used to uniformly distribute the pigments in the glaze. The glaze causes the pigment to adhere to the surface during firing. As exemplified herein, cultured plant cell gums can be used as an emulsifying and suspending agent to produce a glaze of superior consistency, clarity and stability. Further, it has been found that if BLM (Brewers Liquid Maltose) is used as a carbon source during culturing of N. plumbaginifolia, the recovered gum product imports excellent film-forming properties to the glaze. The gum product concentration range in the liquid glaze is between about 0.05 to 3.0% (w/v).

Cultured plant cell gums can also be useful in ceramics forming by plastic extrusion. Completely nonplastic materials can be extruded with the addition of suitable plasticizers such as gums, starches, polyvinylalcohol, waxes and wax emulsions. Grayson, M. (ed.) Kirk-Othmer Concise Encyclopedia of Chemical Technology (1985) p. 237. Cultured plant cell gums can replace prior art gums in such processes. In ceramics forming by slip casting, cultured plant cell gums can be used in the suspension of raw materials to ensure uniform dispersion of the clay and other solid particles in the water.

In cleaning detersive systems, absorption of bath components to the substrate surface may be the most important and fundamental detergency effect. Adsorption is the mechanism whereby the interfacial free energy values between the bath and the solid components (substrate and soil thereon) of the system are lowered, thereby increasing the tendency of the bath to separate the solid components from one another. Surfactant adsorption reduces soil-substrate interactions and facilitates soil removal. Kirk-Othmer Chemical Engineering Encyclopedia, supra, Vol. 22, p. 408. In cleaning detergent manufacturing, the addition of materials to increase viscosity and film-forming properties can enhance surfactant and substrate surface interactions, particularly for vertical surfaces. As exemplified herein, cultured plant cell gums have been found to be useful in improving the viscosity and film-forming properties of detergents. In particular, it has been found that use of BLM as a carbon source in the culturing of N. plumbaginifolia produces a gum product that can impart improved film-forming properties to the cleaning detergent. This is particularly useful for cleaning detergents used to clean vertical surfaces. Detergents can, also contain soil antiredeposition or suspending agents, such as carboxymethylcellulose, polyvinylalcohol and polyvinylpyrollidone. These antiredeposition agents are believed to function by absorbing onto either the substrate or the soil particle, and imparting electrical charges that reduce the affinity between the soil and substrate. Sandford, P. & Baird, J., supra. It is believed that cultured plant cell gums can also function as an antiredeposition agent by coating the substrate and/or soil particles. The gum product concentration range in cleaning detergents is between about 1 and 10% (w/v).

Cosmetic lotions and creams are water-in-oil or oil-in-water emulsions employing emulsifying and stabilizing agents. Emulsifiers, being surface active agents, lower surface and interfacial tensions and increase the tendency of the lotion or cream to spread. A purified acidic heteropolysaccharides obtained from cultured Polianthus has been used in cosmetic creams, lotions, shampoos and cleansing foams. Otsuji, K. et al. EP 0 285 829, published October 12, 1988. As exemplified herein, cultured plant cell gums can be used without prior purification of gum fractions in cosmetic lotions and creams. The gum product concentration range in the cosmetic lotions and creams is between about 0.5 and 4.0% (w/w).

Other applications for cultured plant cell gums include thickeners, emulsifiers or suspending agents for photographic preparations; thickeners for explosives; thickeners and suspending agents for foundry wash coats; thickeners, foam stabilizers and film formers for fire-fighting fluids; emulsifiers and suspending agents for flowable pesticides, suspension fertilizers and animal liquid feed supplements.

The advantages of the cultured plant cell gums over prior art gums include lower production costs, improved purity and improved production reliability. Because the production of cultured plant cell gums does not rely on labor-intensive harvesting of gum exudate from trees (e.g., as is required for gum arabic) or harvesting of seeds or plants for extraction (e.g., guar gum, agar algin, or carrageenan), and can instead be produced under automated conditions, labor costs associated with the production of cultured plant cell gums can be lower. As discussed hereinabove, in agar production, the harvesting of red seaweed is labor intensive in that it is carried out by hand; in some areas of the world, divers in full pressure suits collect individual plants in deep water; in other places, the seaweed can be collected at low tide without the use of diving equipment. Carrageenan or Irish Moss is produced from another red seaweed harvested by raking and hand gathering. Algin is produced from brown algae which can be harvested manually or with small mechanical harvesters. Sandford, P. & Baird, J. (1983) in The Polysaccharides, Academic Press, Inc. Vol. 2, pp. 411-491. Additionally, since production of cultured plant cell gums is carried out in fermentation facilities, production does not rely on weather and is therefore more reliable than prior art gum production. See, for example, Meer et al. (1975) Food Technology 29:22-30. Further, because cultured plant cell gums are produced in fermentation facilities, they can be purer than prior art gums. As discussed hereinabove, because gum arabic is hand collected, it is seldom pure; samples are classified according to grade which depends on color, contamination with foreign bodies such as wood or bark (VanNostrand's Scientific Encyclopedia, supra at p. 1389).

An advantage of cultured plant cell gums over xanthan gum produced by cultured Xanthamonas campestris is that the cultured plant cells do not pose the same cell disposal problem presented by X. campestris, a plant pathogen (Scaad, N.W. (1982) Plant Disease 66(10):882-890). Further, cultured plant cell gums are less expensive than xanthan gum for a variety of applications, including drilling fluids (e.g., Kirk-Othmer Chemical Engineering Encyclopedia (3rd. ed. 1981) 17:153).

A further advantage of the subject gum product is that it can often be used in smaller quantities than prior art gums to achieve comparable effectiveness as an emulsifying, stabilization, suspending, thickening, or gelling agent, as a film forming or coating agent, or as a protective colloid.

All references cited are incorporated herein by reference in their entirety.

The following examples are provided for illustrative purposes only and are not intended to limit the scope of the invention.

### EXAMPLES

### Example 1 - Establishing suspension cultures

### 1.A. - Phleum pratense

Seeds var. Kahu from Hodder & Tolley, seed merchants, 17 Binney Rd. Marayong, Australia, were sterilized by rinsing in ethanol and than soaking 5 minutes in hypochlorite ("chlorize" 1:4). The seeds were then rinsed three times with water and transferred to either liquid or solid medium of Hale et al., supra, containing 2 mg/l 2,4-D.

Suspension cultures were initiated from seeds germinating on either liquid culture or callus culture. In the liquid culture, most seeds germinated after five days. The seed and liquid were chopped in a small sterile blender and then returned to an Erlenmeyer flask and shaken for a further two weeks. The resulting culture was propagated by regular subculturing every 2-3 weeks into suspension culture.

The seeds germinating on agar medium began to form callus immediately. The small calli were dissected off and transferred to fresh agar medium. The calli were subcultured every 3-4 weeks. Initially, the calli are mucoid, but after a number of subcultures, they lose their mucoid appearance. Suspension cultures initiated from mucoid calli produced 2-5 g/l of polysaccharide. Suspension cultures initiated from calli that lost their mucoid appearance and no longer produced polysaccharide.

The suspension medium and procedure were those employed in Hale, A. et al., supra.

Within three days of initiation into the suspension medium, culture filtrates are extremely viscous (i.e., filtrate runs from a 5 ml bulb pipette in about 70-80 seconds, as compared to 15 seconds for water and 16-20 seconds for Pyrus cell culture filtrate). Also, there is very little growth of cells, so the filtrate volume on harvesting is virtually the same as the culture volume (i.e., the packed cell volume is negligible). While polysaccharide production is lost from callus and suspension cultures on repeated subculture, this does not create a problem as it is easy to initiate a new cell line.

### 1.B. - N. plumbaginifolia

Callus was initiated from seeds cultured on 20-30 ml CSV (Gibson et al. (1976) Planta 128:233-239; and Schenk, R. & Hildebrandt, A. (1972) Can. J. Bot. 50:199-204)) medium (below) solidified with 0.5% (w/w) agar. The callus was maintained on the same solid medium, in the dark at 27°C. Maintenance subculturing occurred approximately every 3 weeks. If drying or discoloration of the culture was observed, it was immediately subcultured.

All stock solutions were made up with Milli-Q™ water in glass bottles.

| CS Macro salts | |
|---|---|
| NH₄NO₃ | 24.8 g |
| KNO₃ | 50.1 g |
| (NH₄)H₂PO₄ | 9.2 g |
| CaCl₂·2H₂O | 4.0 g |
| MgSO₄·7H₂O | 8.0 g |

The solution was made up to 1 liter with Milli-Q™ water and stored at 1°C in glass bottles.

| CS organics | |
|---|---|
| Thiamine-HC1 | 100 mg |
| Nicotinic acid | 1000 mg |
| Pyridoxine-HC1 | 100 mg |

The solution was made up to 200 ml with Milli-Q™ water and stored at -20°C in glass bottles.

| CS micro salts | |
|---|---|
| MnSO₄·4H₂O | 6.5 g |
| H₂BO₃ | 2.5 g |
| ZnSO₄·7H₂O | 0.5 g |
| KI | 0.5 g |
| CuSO₄·5H₂O | 100 mg |
| NaMoO₄·2H₂O | 50 mg |
| CoCl₂·6H₂O | 50 mg |

The solution was made up to 500 ml with Milli-Q™ water and stored at -20°C in glass bottles.

| CS Iron solution | |
|---|---|
| Na₂EDTA·2H₂O | 2.0 g |
| FeSo₄·7H₂O | 1.5 g |

The EDTA was dissolved in 60 ml Milli-Q water, while stirring and heating. It was then cooled to room temperature and the FeSO₄·7H₂O was slowly added while also adding NaOH (10 M = 400 g/liter) to keep pH at 5.9. The solution was made up to 100 ml with water and stored at - 20°C in glass bottles.

To prepare one liter of CSV medium, the stock solutions and solids were mixed in the following quantities in approximately 800 ml of Milli-Q™ water:

| | |
|---|---|
| CS Macro | 50 ml |
| CS Micro | 1 ml |
| CS Iron | 1 ml |
| CS Organics | 1 ml |
| Sucrose | 30 g |
| myo Inositol | 1 g |

The pH was adjusted to 5.8 (20-30 drops of 1M KOH). This medium can be modified in various ways without adverse effect, e.g., inositol can be reduced or deleted. The hormone stocks were added in the following quantities:
2.0 ml 2,4-D (stock 1.0 mg/ml)
0.5 ml of kinetin (stock 0.1 mg/ml).
The solution was then made up to 1 liter with Milli-Q™ water and sterilized for 20 minutes at 10 psi (116°C).

Suspension cultures were passaged into fresh CSV medium at 7-day intervals using a 10% inoculum (i.e., 2 ml into 20 ml, 20 ml into 200 ml). Suspension cultures were maintained at a 27°C at a shaker speed of 100 rpm. The cultures were monitored visually for departures from normal color and cell growth patterns. Cultures were also monitored for sterility (i.e., contaminating organisms) and healthy cell morphology (e.g., cell stress).

### 1.C. - Pyrus communis

Callus was initiated from fruit cultured on 20-30 ml BAL (balanced) medium (below) solidified with 0.5% (w/w) agar. The callus was maintained on the same solid medium, in the dark at 27°C. Maintenance subculturing occurred approximately every 4 weeks. If drying or discoloration of the culture was observed, it was immediately subcultured.

All stock solutions for the BAL media were made up using Milli-Q™ water in glass bottles. Vitamins and hormone solutions were stored at -20°C; all other solutions were stored at 1°C.

| Macro elements | |
|---|---|
| NH₄NO₃ | 165 g |
| KNO₃ | 190 g |
| MgSO₄·7H₂O | 37 g |

The Macro solution was up to 1 liter with water.

| Micro elements | |
|---|---|
| H₃BO₃ | 1 g |
| ZnSO4·7H₂O | 1 g |
| MnSO₄·H₂O | 1.44 g |
| NaMoO₄·2H₂O | 0.029 g |
| CuSO₄·5H₂O | 0.0025 g (*) |
| CoCl₂·6H₂O | 0.0025 g (*) |
| The Micro solution was made up to 100 ml with water. | |

| | |
|---|---|
| (*) To obtain 2.5 mg of these salts, 25 mg of each was weighed out in separate containers, and dissolved in 10 ml Milli-Q™; 1 ml of each solution was then used. | |

| Vitamins | |
|---|---|
| Ca pantothenate | 0.1 g |
| myo-Inositol | 10.0 g |
| Biotin | 0.001 g (*) |
| Nicotinic acid | 0.001 g (*) |
| Thiamine-HC1 | 0.1 g |
| Pyridoxine-HC1 | 0.05 g |
| The vitamin solution was made up to 100 ml with water. | |

| | |
|---|---|
| (*) A stock solution containing 1 mg of Biotin + 1 mg of Nicotinic acid per 10 ml was prepared as follows: 10 mg of both vitamins was dissolved in 100 ml of Milli-Q; 10 ml of this solution was used to make up 100 ml of Stock Vitamins. | |

### KH₂PO₄ (potassium dihydrogen orthophosphate)

- KH₂PO₄: 17 g
The solution was made up to 1 liter with water.

### CaCl₂·2H₂O (calcium chloride dihydrate)

- CaCl₂·2H₂O: 6 g
The solution was made up to 100 ml with water.

| Fe·EDTA | |
|---|---|
| FeSO₄·7H₂O | 6.86 g |
| Na₂ EDTA·2H₂O | 9.17 g |

The EDTA was dissolved in 1 liter of Milli-Q™ (magnetic stirrer, room temperature). The ferrous sulphate was dissolved in the EDTA solution. The resulting solution was brought to a boil, cooled and stored in screw capped glass bottle at 1°C.

### KI (potassium iodide)

- KI: 0.03 g
The KI was dissolved in 20 ml Milli-Q.

### 2,4-D (2,4-dichlorophenoxyacetic acid) 0.1 mg/ml

- 2,4-D: 50 mg
The 2,4-D was dissolved in 5 ml of commercial grade ethyl alcohol (95%). The 2,4-D was injected slowly under the surface of 495 ml of Milli-Q™ water, using a Pasteur pipette and a magnetic stirrer.

To make up the BAL medium, the concentrated stock solutions and solids were mixed (magnetic stirrer) in the quantities indicated below and water added to approximate 900 ml.

| | |
|---|---|
| Macro elements | 10 ml |
| Micro elements | 1 ml |
| Vitamins | 1 ml |
| KH₂PO₄ | 10 ml |
| CaCl₂ | 2.5 ml |
| Fe·EDTA | 2.5 ml |
| KI | 0.5 ml |
| 2,4-D | 10 ml |
| L-Asparagine | 180 mg |
| L-Ascorbic acid | 50 mg |
| Thiourea | 25 mg |
| Sucrose | 40 grams |

The pH was adjusted to 5.8 - 6.0 with KOH (0.1 or 1M). The final volume was adjusted to 1 liter with water. For solid medium, 0.5% (5 g/liter) agar was added after adjusting pH and volume. The final medium was sterilized for 20 minutes at 10 psi (116°C).

Suspension cultures were passaged into fresh BAL medium at 14 day intervals using a 20% inoculum. The cultured were maintained at 27°C at a shaker speed of 100 rpm. Cultures were monitored for sterility, cell morphology, and departures from normal culture color and cell growth. After subculturing into fresh BAL medium, the packed cell volume (PCV) of the old culture is measured to assess whether the culture conditions are successfully maintaining the cell line in a stable growth pattern. If the PCV declined progressively over several subcultures, the cell line was revived with a single passage on double phosphate BAL medium.

### 1.D - Enhanced polysaccharide production using BLM

When BLM was used as a carbon source to enhance polysaccharide production by Nicotiana or Pyrus, it was typically used at a culture medium concentration of between about 80 to 200 g/liter of medium, or preferably at about 162 g (wet weight) per liter of medium.

### Example 2 - Recovery of Gum Product from Cultured N. plumbaginifolia

N. plumbaginifolia whole broth was harvested from a fermenter. The whole broth was filtered using a filter having a pore size of about 100 µm. The filtrate was then heated to 80°C for 30-60 minutes to denature enzymes in the filtrate. The filtrate was then cooled. Complexant (e.g., Na₂EDTA·2H₂O; 1 g/l) was added either prior to filtration, after filtration and prior to heating, or after cooling.

In some cases, the filtrate was stored prior to further processing. When storage time was longer than 18 hours, preservatives, 1.0 g/l potassium sorbate and 0.34 g/l sodium metabisulfate, were added. These preservatives allowed storage at ambient temperatures (15°-25°C) in sealed containers for prolonged periods.

The filtrate, warmed to 30-80°C to reduce viscosity, was next concentrated by ultrafiltration (10,000 MW membrane, Amicon Model DC10LA) to about 20-25% of its original volume or until viscosity made further significant concentration difficult. The concentrate was then diafiltered using the same membrane with five equal volumes of distilled H₂0, and concentrated again by ultrafiltration to the point at which viscosity or gelling inhibited further progress.

Where the gum product was intended to be used in industrial compositions such as in drilling mud, adhesives, cleaning detergents, dyestuffs, paper, acrylic resin and oil emulsion paints, or printing ink, the concentrate was directly spray dried (Niro Production Minor, Niro Atomizers, Denmark) using a 200°C inlet temperature and a 100°C outlet temperature.

Where the gum product was intended to be used in foods, pharmaceuticals, or cosmetics, the concentrate was further purified by an alcohol precipitation method comprising a precipitation and washing step. The concentrate was chilled to 1-4°C, and NaCl or KCl was added as a concentrated solution, followed by slow addition with stirring of 2-4 volumes cold (1-4°C) ethyl or isopropyl alcohol. The NaCl or KCl was added in an amount to give a concentration of 0.03-0.1% w/v in the alcohol-containing mixture. The mixture was allowed to stand at 1-4°C for 1-18 hours and then filtered using 2-4 layers of surgical gauze. The filtrate was washed in 67-80% alcohol at 1-4°C and the wash was removed by filtration using 2-4 layers of surgical gauze. The alcohol can be recovered and recycled by distillation.

Where further purification was desired, the alcohol purification procedure was repeated one or more times. A variation of the purification procedure comprises repeated precipitation and filtration steps without intervening washing steps.

The purified material was then directly drum dried (Blaw-Knox Co. Buffalo, NY). Alternative drying methods are fluidized bed, vacuum tumble drying and "flash-spin" drying. The purified material can also be spray-dried or freeze-dried if first rehydrated with 1-2 volumes distilled H₂0. Example 3 - Functional Assessment of Recovered Gum Products

### 3.A. - Emulsion Testing: Measurement of Droplet Size, Turbidity and Stability

A comparison of the emulsifying properties of Pyrus gum and a prior art gum, gum arabic, was conducted to determine whether the claimed gum has emulsifying qualities comparable or improved relative to the prior art gum. Aqueous solutions of Pyrus gum and gum arabic were mixed with D-limonene oil to produce emulsions, which were then tested for droplet size, turbidity and shelf life stability.

In order to clarify or reduce complexing of the pectic fraction of the Pyrus gum prior to use, 5 grams of Pyrus gum were dissolved in 500 ml of distilled water and boiled for 5 minutes. Concentrated EDTA solution was added until the insoluble pectic material was dissolved. The solution was filtered through two layers of Whatman glass fiber filter paper GF/F under vacuum and dialyzed (MW cutoff 14,000-16,000) against distilled water at 4°C for 24 hours. The volume of the solution was then reduced under vacuum by rotary evaporation and freeze dried. Gum arabic was obtained from Sigma, No. G-9752. D-Limonene (p-mentha-1,8-diene) was obtained from Bush Boake and Allen.

Stock solutions of gum arabic (250 mg/ml) and Pyrus gum (62.5 mg/ml or 12.5 mg/ml) were pipetted in duplicate to give final concentrations of 0, 0.2, 0.5, 1, 5, 10 and 20% (w/v). The Pyrus solution could not be prepared at concentrations greater than 5% (w/v) due to its viscosity and gelling properties. Twenty percent D-limonene oil in water emulsions were prepared by injecting the oil into the aqueous solutions under the surface of the solutions while being mixed in an Ultraturrax (Ystal T1500, 25-240V, West Germany) at setting 4 for 15 seconds. The speed of the Ultraturrax was increased to setting 7 for 45 seconds to produce the cloud emulsion. The emulsions were allowed to stand for 0.5 hours to allow bubble dispersal.

To determine emulsion capacity, droplet size, turbidity and shelf-life were measured for each emulsion. Emulsion capacity increases with decreased droplet size, increased turbidity and increased shelf-life stability. The droplet size of the cloud emulsion was examined microscopically by placing 2 drops of the emulsion on a slide and diluting with 2 drops of water and estimating droplet size using a calibrated eye piece graticule. Cloud turbidity was measured by diluting duplicate 5 µl aliquots of cloud emulsion into 5 ml of 0.1% (w/v) sodium dodecylsulphate and measuring absorbance at 500 nm. Cloud emulsions were tested for shelf-life stability by centrifuging at 2,500 rpm for 10 minutes and observation of the resulting separation of oil and water phases. The results are set forth in Table 1:

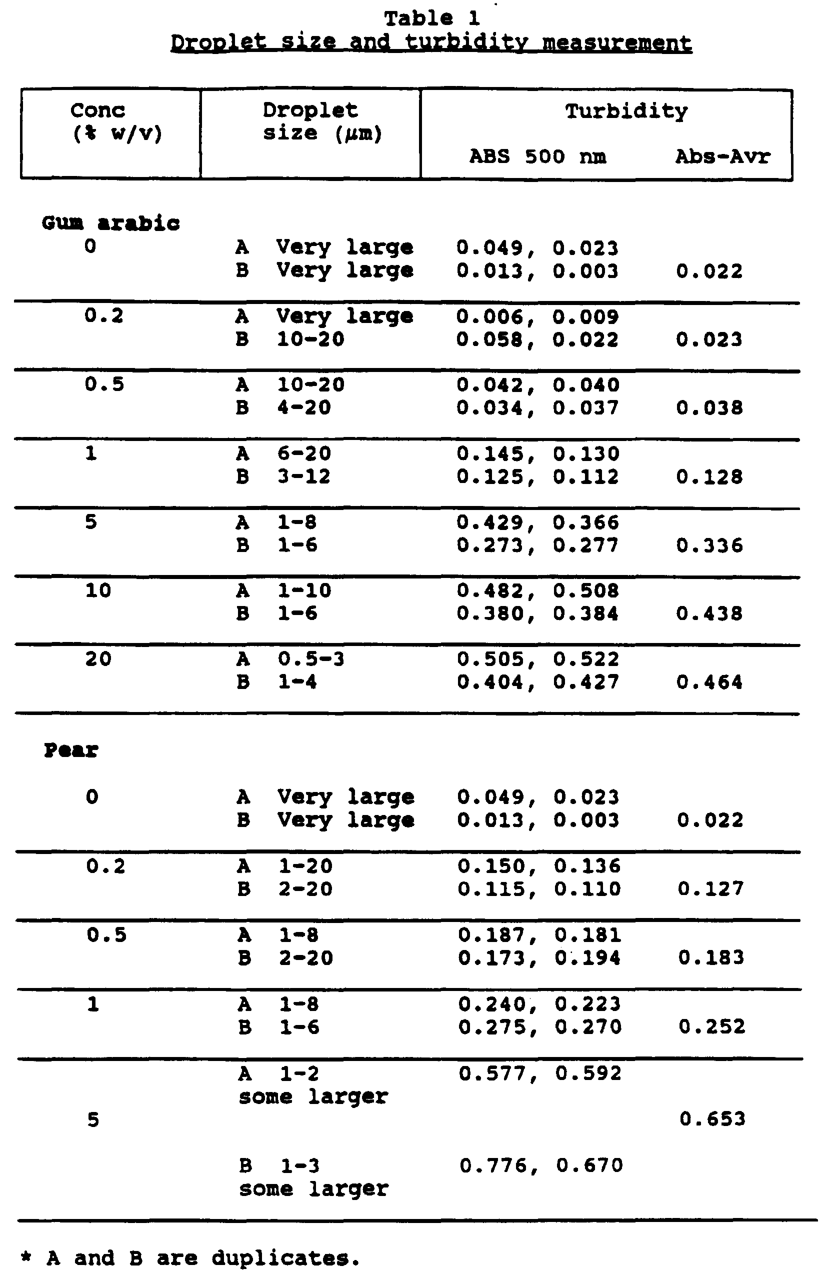

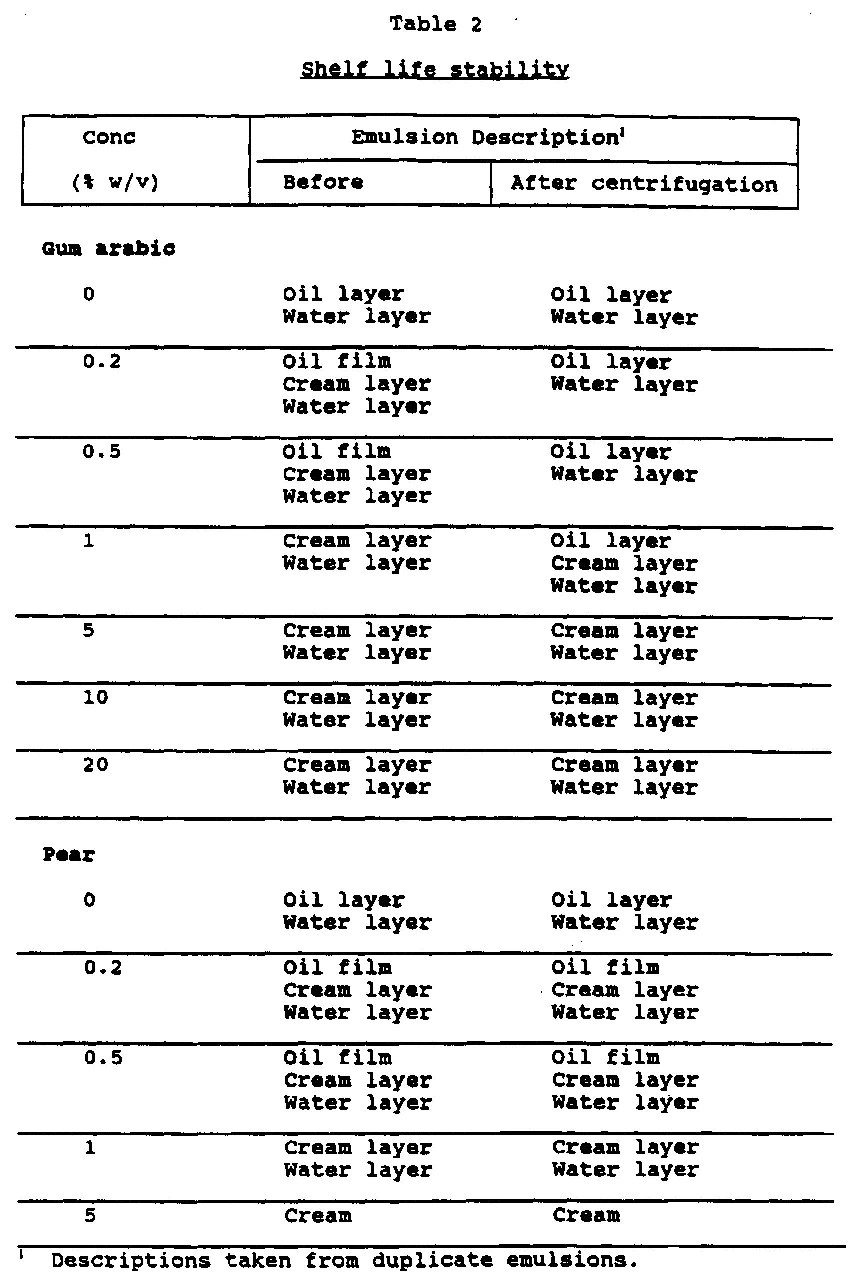

From the foregoing results, it is seen that when emulsifying 20% D-limonene in water, Pyrus gum on a weight for weight basis produces smaller droplets at a lower concentration than gum arabic. For example, at 0.2% (w/v) of Pyrus gum, the emulsion mixture has a film of free oil, a cream layer stable to centrifugation, oil droplets of 1-20 µm and a cloud turbidity at 500 nm of 0.127. In contrast, 0.2% (w/v) gum arabic in an emulsion mixture has an unstable cream which separates completely to oil on centrifugation, has a larger droplet size (10-20 µm) and an average cloud turbidity reading of 0.023 at 500 nm. Those results indicate that the Pyrus gum his improved emulsifying qualities relative to those of gum arabic at the same concentration.

Emulsion stability can also be assessed by the following method. An oil-in-water emulsion was produced with a range of gum product concentrations (e.g., 0.2, 0.5 and 0.7 % (w/v)):

| | | | |
|---|---|---|---|
| gum (g) | 0.1 | 0.25 | 0.35 |
| oil (ml) | 10.00 | 10.00 | 10.00 |
| water (ml) | 40.00 | 40.00 | 40.00 |
| Total | 50.00 | 50.00 | 40.00 |

The gum product was dissolved in water using the ultraturrax (John Morris Scientific Equipment) at a setting of 4. Oil (Crisco, polyunsaturated blend) was then added while mixing and held at setting 4 for 45 seconds. The solution was further mixed at setting 8 for 45 seconds. The emulsion obtained was poured into 50 ml measuring cylinders (21 mm internal diameter), sealed with aluminum foil and stored at 27°C. It was then observed for up to a week. Creaming or separation was expressed in percentage volume.

The volume of oil can be varied to provide an HLB in the emulsion that is typical for the intended application.

For measuring the stability of a water in oil emulsion comprising a cultured plant cell gum, ASTM method 3707 or an adaption thereof can be used.

### 3.B. - Viscosity Testing

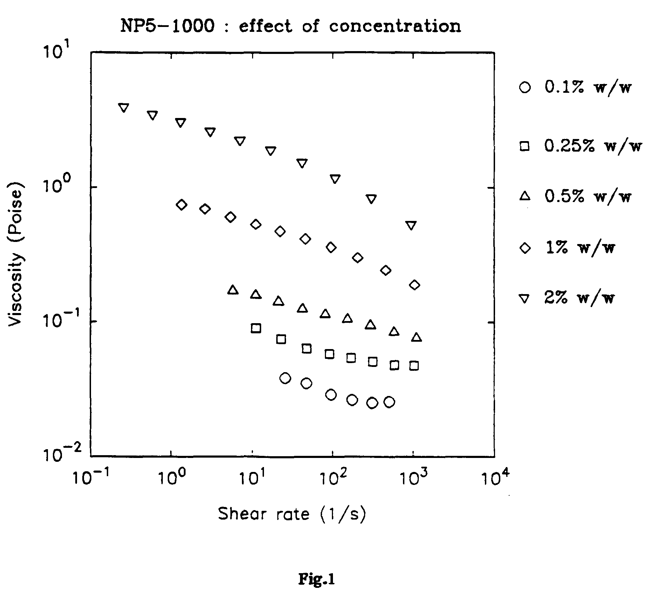

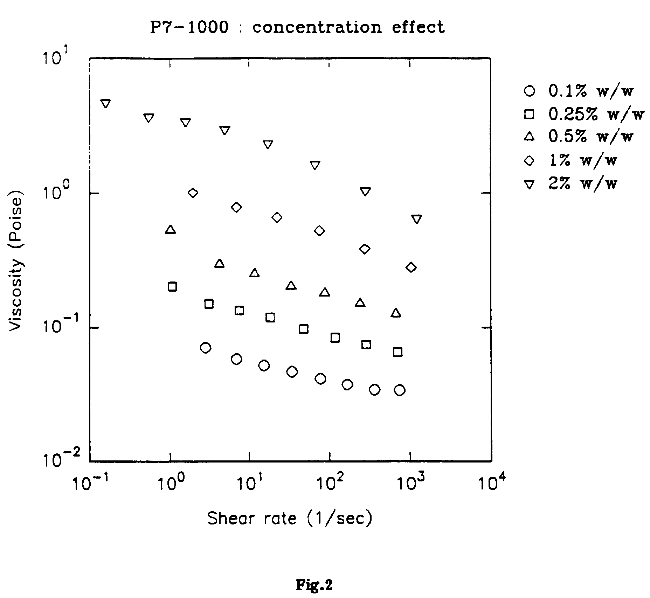

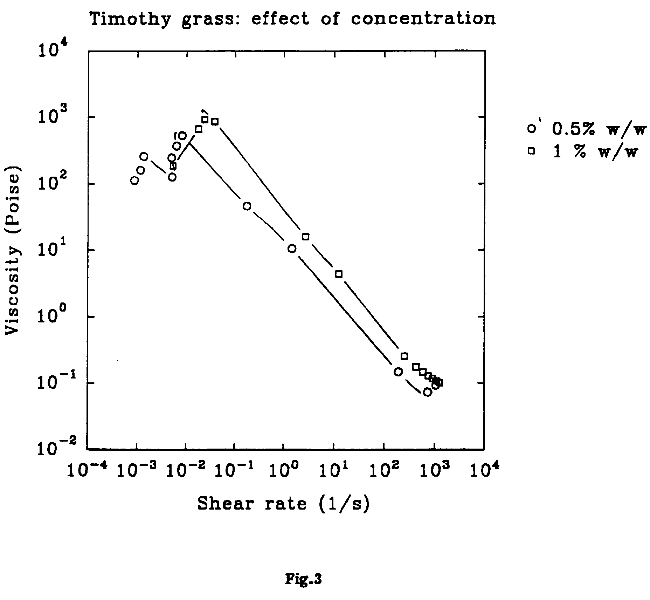

The flow behaviour of the gum in aqueous solution or mixtures was assessed over a range of gum concentrations, pH, temperature and shear rates. The gum product was dissolved in water with stirring and heating to 60°C. Viscosity was measured on the Carri-Med Constant Stress Rheometer (CSL 100) using a cone and plate measurement system with 2° cone angle, 56µ measuring gap and a 227.8 dynes cm/s² system inertia. Shear stress was 0-800 dynes/cm². The effect of gum concentrations and pH was measured at 20°C. Results were expressed as viscosity (Poise) as a function of shear rate (1/s). Figures 1 and 2 are exemplary plots of viscosity as a function of shear rate for a range of gum concentrations from 0.1% (w/w) to 2% (w/w) for *N*. *plumbaginifolia* (NP5-1000) and *P*. *communis* (P7-1000), respectively. Figure 3 plots viscosity vs. shear rate of Timothy grass gum at 0.5% w/w and 1% w/w. In all cases, viscosity increases with gum concentration and decreases, displaying a shear thinning effect, with increasing shear rate. Thixotropic profiles indicate if a gum is suited for particular applications where shear thinning is required, e.g., for use in drilling muds. Timothy grass displays structured viscosity at low shear rates. The presence of such structure indicates that a gum is suitable for applications where yield stress is required, e.g., for suspension of titanium dioxide in paint.

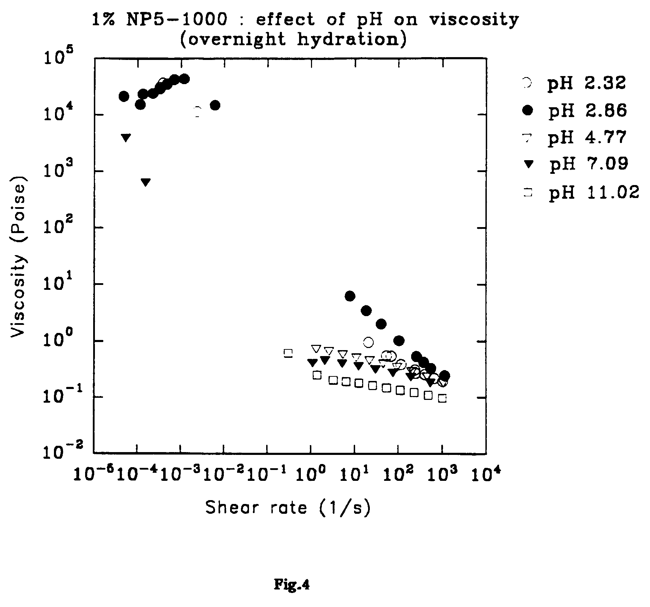

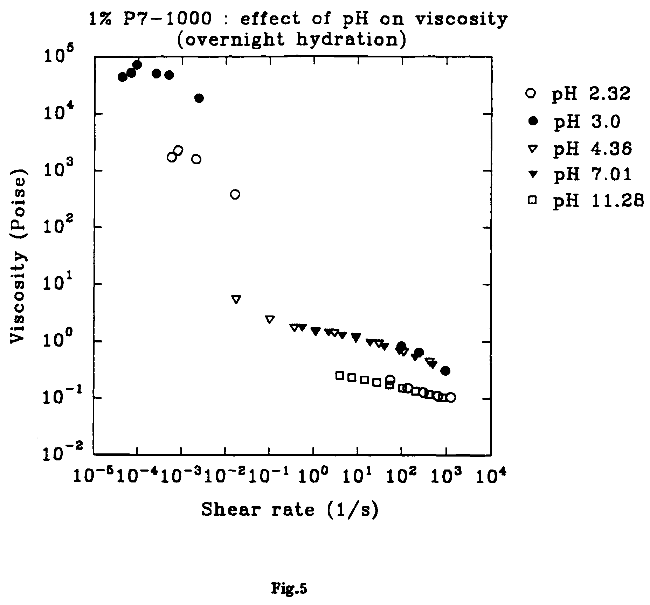

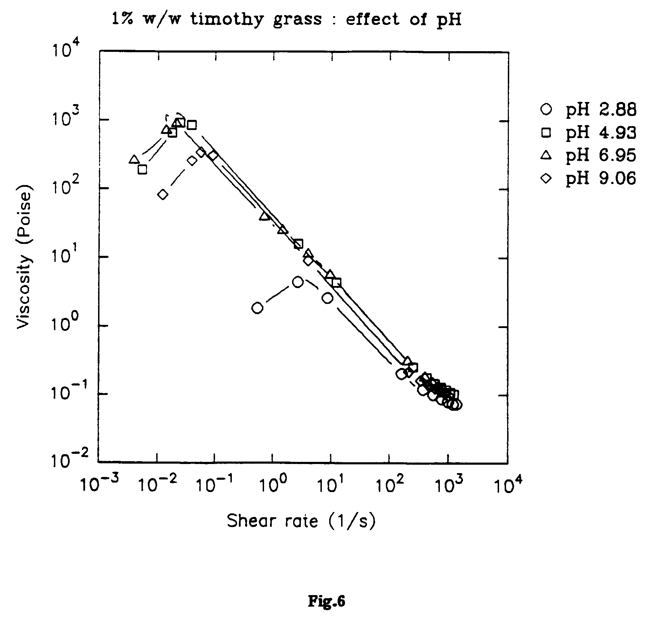

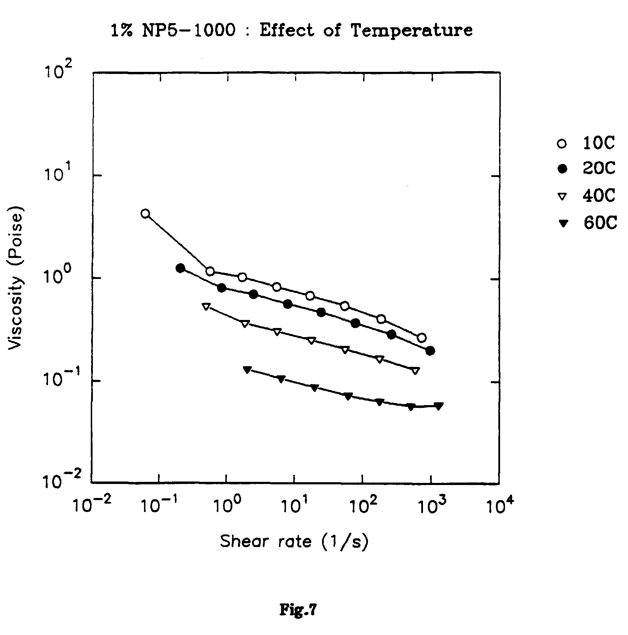

Figures 4-6 are exemplary plots of the effect of pH on viscosity for *N. plumbaginifolia* (NP5-1000, 1% w/w), *P*. *communis* (P7-1000, 1% w/w) and Timothy grass (1% w/w). This data indicates the suitability of a gum as a viscosifier or thickener over the operating pH range of a given application. Figure 7 is an exemplary plot of the variation of viscosity as a function of shear rate for temperatures ranging from 10°C to 60°C. This data indicates the suitability of a gum as a viscosifier or thickener over the operating temperature range of a given application.

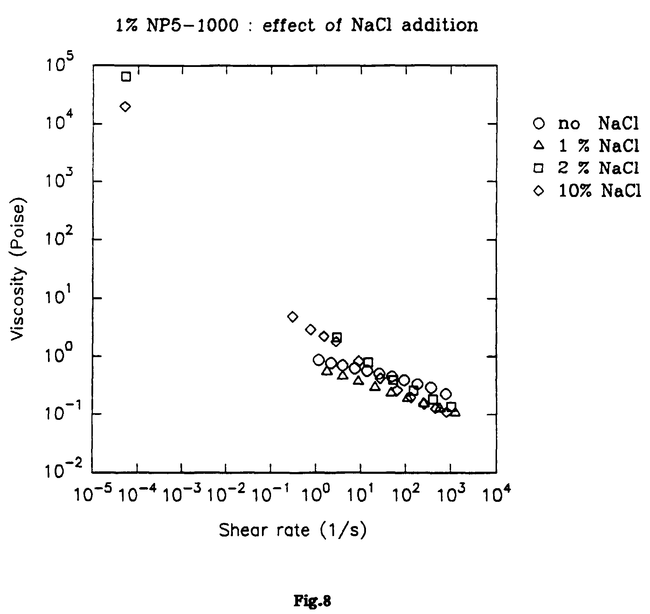

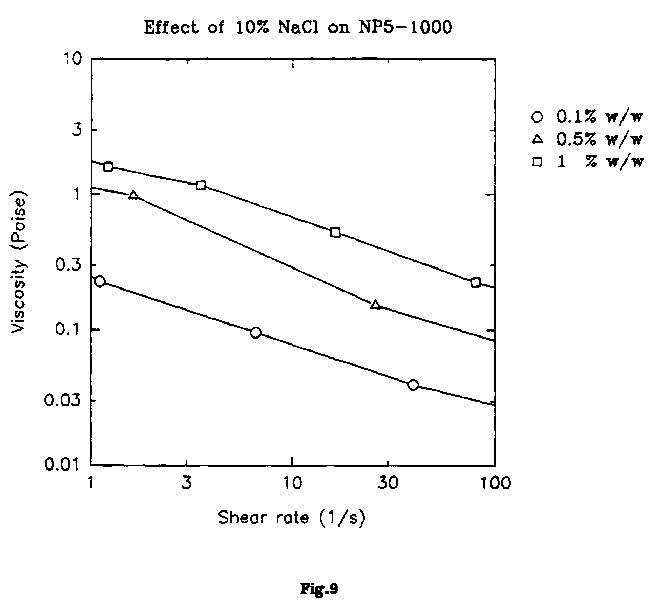

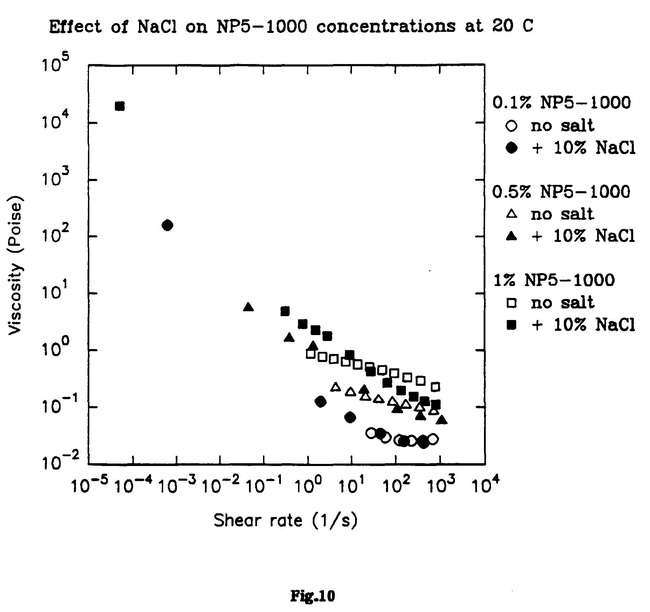

Viscosity of the plant gum products is relatively unaffected by addition of salt as shown in Figures 8-10. This data indicates the suitability of a gum for use in offshore oil and gas well operations.

### 3.C. - Gel Strength Testing

Gel strength is assessed by measuring rupture strength, shear modulus and back extrusion. Back extrusion is of particular interest because it can distinguish between and characterize soft gels and viscous fluids.

### 3.C.1 - Rupture Strength

Rupture strength is the force required to compress and rupture a gel sample. For rupture strength, the force is proportional to the sample weight.

The gel samples were prepared by mixing *P. communis* gum (0.2-0.5% (w/v)) or *N.plumbaginifolia* (0.5-1.0% (w/v)) in water in 50 mm plastic petri dishes and storing them at 15°C overnight. Rupture strength was measured by compression on the Instron 1122, using a probe of 150 mm in diameter at a cross-head speed of 50 mm/min.

### 3.C.2 - Shear Modulus

Shear modulus is a measure of the force required to shear/cut the gel. Shear modulus is expressed as stress divided by strain. For shear modulus, the force is proportional to the sample weight.

The gel samples were prepared as in C.1 in a 24 mm diameter glass vial and stored at 15°C overnight. Shear force was measured on a modified puncture strength meter (Oakenfull, D.G. et al. (1987) "A method for determining the absolute shear modulus of a gel from a compression test" in Gums and Stabilizers for the Food Industry, Vol 4, Phillips, G.O. et al. (eds.) IRL Press, Oxford) with a probe of 3 mm in diameter at the cross head speed of 5 mm/min for 20 seconds. Shear modulus was then calculated using a mathematical model set forth in Oakenfull, D.G. et al. (1987)..

### 3.C.3 - Back-extrusion

Back-extrusion force is the force required to compress and shear a gel sample. In back-extrusion, force is independent of sample weight.

The gel samples were prepared as in C.1 in 200 ml beakers of 64 mm and stored at 15°C overnight. Back-extrusion was performed on the Instron 1122 by plunging a probe of 60 mm in diameter at a speed of 100 mm/min to a depth of 50% into the gel.

### 3.C.4. - Effective Temperature Range of Gel: Determination of melting and setting points

The melting point is determined by observing the temperature at which a 10 ml gel begins to melt in a 11 mm diameter spectrophotometric tube. The determination was aided by observing small glass beads (0.08 g) sinking into the melting gel. AS there can be temperature gradients within the gel, a melting range can be observed. The experiment was carried out in a Thermoline waterbath in 5°C steps.

The setting point was determined by observation of gelling in spectrophotometer tubes. The gel samples in the tubes were stored overnight (18 hours) at a range of temperatures, and the tubes were thin inverted to observe if setting had occurred. The temperatures tested were 6°, 10°, 15°, 20°, 25°, 27°, 30°, 37.2° and 45°C.

### 3.D. - Encapsulating Capacity

Encapsulating capacity can be assessed by evaluating a gum-containing spray-dried emulsion in terms of flow characteristics and stability as described below.

Dry powdered drink mixes are prepared by spray-drying flavor oil emulsions. Flavor emulsions are produced according to the formulations given below:

| Ingredient | Control Mix | Gum Mix |
|---|---|---|
| Maltodextrin (Fielder's PHS 17) | 63.7 g | 91.0 g |
| Encapsulating Agent (N-Lok or gum to be tested) | 27.3 g | 0.7 g |
| Orange (or other flavor) Oil | 20.0 g | 20.0 g |
| EDTA | -- | 0.35 g |
| Water | 350 ml | 350 ml |

The chelating agent EDTA may be included in the gum mix to assist in dissolution of the gum from a freeze dried powder.

The N-Lok encapsulating agent refers to a starch product produced by the National Starch Company, Australia. N-Lok is currently used as an encapsulating agent for spray-dried flavor oils used in such products as jelly crystals, dessert mixes, cake mixes, drink mixes, packet soup mixes, snack foods, savory dips, and spreads.

The control mixture is prepared by dissolving the encapsulating agent N-Lok and maltodextrin in water prior to the addition of orange oil.

To avoid a possible interaction between the gum and the maltodextrin, the gum mixture is prepared by adding the oil to the hydrated gum before the addition of the maltodextrin solution. The gum is dissolved in about 200 ml of the water by stirring on a magnetic stirrer and warming to 50-60°C. The balance of the water is used to dissolve the maltodextrin and the EDTA, using a magnetic stirrer at room temperature. The maltodextrin/EDTA solution is then added to the gum solution and the oil added while stirring using a Silverson High Speed Mixer. The mixes are spray-dried in a "baby" Niro Spray Drier, using an inlet temperature of approximately 200°C and an outlet temperature of 100°C. The spray-drying and control mixing methods are the standard procedures used by Bush Boake Allen in manufacturing spray-dried encapsulated flavor oils. Flow characteristics and bulk densities are compared.

The control and gum spray-dried powders may be used in powdered drink formulations, e.g., as follows:

### A. Commercial orange drink powder formulation.

| Ingredient | Percent (w/w) |
|---|---|
| Caster sugar | 94.5 |
| Citric acid | 3.0 |
| Xanthan gum | 0.4 |
| Sunset Yellow | 0.05 |
| Spray-dried orange flavor powder (containing either gum 0.0126 g or N-Lok 0.49 g per 2 g of powder) | 2.0 |

### B. Commercial drink formulation commonly used for taste testing.

| Ingredient | Percent (w/w) |
|---|---|
| Sugar | 8. |
| Citric acid | 0.008 |
| Spray-dried orange flavor powder (containing either gum 0.0063 g or N-Lok 0.245 g per 0.1 g of powder) | 0.1 |
| Water | 91.82 |

The drink mixes are evaluated by a panel of tasters who are asked to scale the intensity of flavor in unidentified samples presented in random order. Statistical analysis of the data is done to determine perceived intensity of the flavors among the drink mixes, such that the encapsulation properties of the gum may be compared to those of N-Lok.

To assess the stability of the spray-dried powders, the oil content of the control and gum powders may be determined after a period of time, e.g., 3 months and again at 4 months storage at room temperature. A further period of 48 hours at higher temperatures, e.g., 65°C, may also be used.

### 3.E. - Adhesive capacity

Adhesive capacity can be measured by using standard methods such as ASTM (American Society for Testing Materials) method D1713 ("Bonding Permanancy of Water- or Solvent- Soluble Liquid Adhesives for Automatic Machine Sealing Top Flaps of Fiberboard Specimens") and D1581 ("Bonding Permanancy of Water- or Solvent-Soluble Liquid Adhesives for Labelling Glass Bottles"), or adaptations thereof.

### Example 4 - Papermaking: Preparation of Paper Hand Sheets

A superior strength paper can be produced using the procedure described in Australian Standard 1301 APPITA P203s/80 by adding N. plumbaginifolia gum product at the wet end to improve the physical properties of the dry sheet. The observed improvements include increased paper strength (both burst and tensile), greater resistance to erasure, reduced "fuzz" or lint on the paper surface and reduced rate of water penetration as compared to hand sheets prepared without a gum beater aid. The gum product allows for a retention of wet strength and improved yield by providing a more uniform distribution of fines.

The following method (Australian Standard 1301 APPITA P203s/80) was used for the preparation of hand sheets:

Commencing with wood fibre pulp (sourced as chemically treated pulp, semi-chemical pulp, or mechanical pulp or recycled pulp), the gum product was dissolved in a quantity of water sufficient to produce a 2% solids solution. One liter of the dissolved solution was added to 4 liters of pulp placed in a container. Adequate mixing was ensured by sparging for at least 15 minutes. A sample of 500 ml was then place into a larger tapering 15 liter vessel with a 60 mesh screen at the base 100 mm in diameter. A further 10 liters of processed water was added and the mixture was sparged from the base of the vessel for at least 15 seconds to ensure thorough mixing. The base valve was then opened, allowing processed water to drain away, retaining all of the fibers on the wire mesh screen. The base screen was removed from the unit base and covered with a blotter, allowing the wet fibrous mat to be retained by the blotter. Successive cycles produce a number of samples which are then stacked and pressed in a stack to remove excess water. They were then placed in a drying cabinet and maintained at a standard 23°C, 50% relative humidity until testing.

Testing revealed that the subject gum-containing paper has superior tensile strength, stretch, work to rupture and extensional stiffness on an Alweitron Universal Testing machine. Methods for testing paper are known in the art and include, e.g., Australian Standard 1301.403s-89 for "Bursting Strength of Paper;" Australian Standard Appita P404s-81 for "Tensile Strength of Paper and Paperboard;" Australian Standard 1301.419s-89 for "Water Vapour Transmission Rate of Paper;" Australian Standard 1301.411s-89 for "Water Absorptiveness of Paper and Paperboard (Cobb Test);" and Australian Standard Appita P406m-86 for "Bending Quality of Paperboard."

### Example 5 - Adhesives: Preparation of re-moistenable adhesive

A satisfactory adhesive for envelopes, labels, stamps and aluminum foil sheets, which is of the water re-moistenable type, was prepared as follows:

| | | |
|---|---|---|
| 1. | N. plumbaginifolia (BLM carbon source) | 1000 gm |
| 2. | Sodium Chloride | 20.5 gm |
| 3. | Glycerol | 20.5 gm |
| 4. | Potato starch | 20 gm |
| 5. | Water | 1300 ml |
| 6. | Preservative | 1 gm |

The water was placed in a high speed mixer and mixing was begun at a slow speed. The gum product was slowly added, allowing it to fully dissolve in the mixing process. After 4 minutes, the sodium chloride, glycerol, starch and preservative were added. After thorough mixing, the mixture was left to stand for 1-1/2 hours.

This produced an adhesive which was applied to the surface of paper and dried. It remained inactive until moisture was reapplied. It was found to be a superior gum for use in these applications as it has good affinity for water and does not cause discoloration of the paper or become brittle on aging. It was found that the adhesive glued pieces of aluminum foil to paper very firmly and also glued pieces of paper together in a manner similar to commercial adhesive pastes.

### Example 6 - Oil and Gas Well Applications: Preparation of Drilling Mud

A satisfactory drilling mud or fluid can be prepared in stirred tanks as follows:

A large 1,000 liter tank was filled with water. About 6% by weight bentonite (montmorillonite) was added while stirring slowly and continuously until dissolved. In a second 1,000 liter tank filled with water, about 3% by weight N. plumbaginifolia gum product was added while stirring slowly until dissolved. In a third holding tank, equal quantities of gum product mixture and bentonite mixture were mixed. This produced a basic drilling fluid to which was added up to 30% solids of barium sulphate or 30% chalk as weighting agents depending upon the nature of the surrounding rock structure. If desired, a biocide can be added to prevent fermentation during storage or down-hole.

The resulting drilling fluid has increased viscosity, and can provide an improved flow of material from the bit to the surface and a uniform dispersion of the solids, thereby acting as a protective colloid. It can also lubricate and reduce fluid loss into porous rock. The fluid retention properties of the plant gum product has been treated using a 35 mm diameter Whatman No.2 filter paper under vacuum. The rate at which water flowed through the filter was tested at 650 ml/minute. A 0.5% solution of *P.communis* gum was similarly tested under the same conditions and yielded an average filtration rate of 5 ml/minute over a 30 minute period. Observation of the filter disc showed very little build-up of colloidal material. This indicated that aqueous solutions of low concentrations of the plant gum would likely advantageously produce thin filter cakes, as well as low fluid loss, during drilling operations in porous rocks.

The resulting drilling fluid is particularly efficacious in providing a uniform suspension and maintaining a consistent fluid in drilling through shale layers, broken rock that has been stabilized, or magnesium or calcium containing rock. During cementing, a stabilizing fluid containing the subject gum product will also have reduced fluid loss. The insensitivity of the viscosity of solutions containing the plant gum product to salt makes them particularly suitable for use in offshore oil and gas well operations.

The N. plumbaginifolia gum product, when in an aqueous dispersion with calcium, possesses galling properties. Such gelling properties can enhance suspension of solids in a drilling mud even when flow has stopped.

### Example 7 - Printing Applications

### 7.A. Preparation of Printing Ink

A satisfactory emulsion or suspension water-based flexo ink for printing was prepared using the N. plumbaginifolia gum product as a suspension agent to provide uniform dispersion of the pigment elements and prevent the ink from separating. To a typical ink formulation of:
1. Carbon Black
2. Mineral Oil
3. Sodium silicate
4. Sodium carbonate
5. Water
was added about 2% by weight of gum product to produce a fine uniform stable suspension of the solid ingredients. Using a high speed mixer running at low speeds the gum product was added to the mixture until thoroughly dispersed. The emulsion mixture was left to stand for 1-1/2 hours prior to use.

### 7.B. Preparation of a Lithography Fountain solution

The N. plumbaginifolia gum product provides a satisfactory substitute for gum arabic in several lithography solutions or mixtures including the plate sensitizer solution, the fountain solution and the protecting solution (used during plate storage). The gum product imparts good wettability particularly to the fountain solution. It also supplies the viscosity required to allow the fountain solution to cling to the plate without running off or forming isolated droplets or pools on the plate.

A fountain solution was prepared as follows:

| | | |
|---|---|---|
| 1. | Water | 700 ml |
| 2. | Propylene Glycol | 50 ml |
| 3. | Biocide Parabens (methyl/ethylhydroxy parabenzoic acid at 0.5-2.0% (w/v) in water adjusted to pH 7.0 with phosphate buffer | 1 ml |
| 4. | gum product solution 3% (w/w) | 200 ml |
| 5. | pH buffer | 40 ml |

All ingredients other than the gum product solution were added to a mixture, and stirred until dispersed (10 mins). The gum product solution was then added, and stirring was continued.
The mixture was then allowed to stand for 30 minutes before use.

### 7.C. Comparison of N. plumbaginifolia gum to gum arabic in fountain solutions

The following formulae were made up by Cetec Pty. Ltd., a consultant. All values (except pH) are w/w percent.

| | F1 | F2 | F3 | F4 |
|---|---|---|---|---|
| Water | 70 | 90 | 70 | 90 |
| Propylene Glycol | 5 | - | 5 | 5 |
| 3% w/w gum arabic soln. | 15-20 | - | - | - |
| biocide | 0.1 | 0.1 | 0.1 | 0.1 |
| pH buffer (phosphate) | pH 5-7 | pH 5 | pH 5-7 | pH 5 |
| Phosphoric acid | - | 2 | - | 2 |
| gum arabic EDTA | - | 2 | - | 2 |
| EDTA | - | 0.5 | - | 0.5 |
| N. plumbaginifolia gum 3% (w/v) | - | - | 0.3-0.4 | - |
| N. plumbaginofolia gum | - | - | - | 0.3-0.4 |
| F1 and F2 are standard fountain solutions that employ gum arabic. F3 and F4 are identical to F1 and F2, respectively, except that N. plumbaginifolia gum product has been substituted for the gum arabic in a weight that is 1/50 of the gum arabic weight. | | | | |

When these fountain solutions were employed in an offset litho printing, it was found that the N. plumbaginifolia gum product performed comparably to the gum arabic fountain solutions in terms of ink-plate roll up and in degree of plate background desensitization. The plate wetting characteristics of the two products were also very similar. The N. plumbaginifolia gum was found to be less soluble in isopropyl alcohol than gum arabic; since isopropanol is very widely used as part of the dampening system of modern, fast lithographic offset presses, this may be a negative feature.

### Example 8 - Fabric Printing: Preparation and use of reactive dyestuff for wool or cotton

Satisfactory dyeing of wool and cotton was accomplished as follows:

First, a thickening was prepared:

| | | |
|---|---|---|
| 1. | N. plumbaginifolia gum product | 150 gm |
| 2. | Cold water | 2800 ml |
| 3. | Sodium metaphosphate (Calgon™) | 30 gm |

The water was agitated with a high speed mixer during gradual addition of the Calgon™. The gum product was then added slowly, but fast enough so that all the powder was added before the viscosity has risen appreciably. Stirring was continued for 5-10 minutes until all particles were swollen and had formed a thick suspension. The mixture was allowed to stand for 1-1/2 hours.

Then the following were added:

| | | |
|---|---|---|
| 4. | Diphasol™ solution | 115 ml |
| 5. | Hot water | 975 ml |
| 6. | White spirit | 3750 ml |
| 7. | Resist salt L™ | 150 gm |

The thickening mixture was then stirred in the high speed mixer for 20 minutes.

The screen printing paste was prepared by mixing the following:

| | | |
|---|---|---|
| 1. | Dyestuff | 3 gm |
| 2. | Urea | 10 gm |
| 3. | Hot to boiling water | 30 ml |
| 4. | Thickening (as above) | 50 gm |
| 5. | Sodium bicarbonate | 4 gm |

Using a high speed mixer, the dyestuff and urea were thoroughly dry mixed. Then the hot water and thickening were added and mixed.

The printing paste was used in a standard fabric screen printing method. The printed cotton and wool were then dried followed by steaming for 8 minutes. They were then rinsed thoroughly in cold water followed by a soaping at or near the boiling point with a detergent solution of Lissapol ND (2% w/w solution) and finally rinsed in cold water. The printing on the wool and cotton material appeared stable.

### Example 9 - Paints

### 9.A. Preparation of Acrylic Resin Paint

A stable water emulsion was prepared using the following formulations:

Premix in a ball mill:

| | | |
|---|---|---|
| 1. | Tap water | 125 ml |
| 2. | Daxad 30™ Dispersant | 8 gm |
| 3. | Tergitol NPX™ Surfactant | 4 ml |
| 4. | Victawet 35B™ Wetting Agent | 2.5 ml |

Then the mill speed was increased and the following was added slowly:

| | | |
|---|---|---|
| 5. | Chemacoil TA-1001™ Resin | 74 gm |

The speed was adjusted to disperse the following pigments and additives:

| | | |
|---|---|---|
| 6. | Zinc oxide AZO-ZZZ-33™ | 75 gm |
| 7. | Titanox RANC™ Rutile Titanium Dioxide | 175 gm |
| 8. | Titanox A168L0™ Anatase Titanium Dioxide | 25 gm |
| 9. | Asbestine 3X™ Talc | 100 gm |
| 10. | Ethylene Glycol | 18.5 gm |
| 11. | Nuodex PMA-18 Mildewoide™ | 3 gm |
| 12. | Nopco NDW™ Defoamer | 4 gm |

The mill was then slowed to mixing speed.

| | | |
|---|---|---|
| 13. | N. plumbaginifolia gum product emulsion (2% w/w aqueous solution) | 165 ml |
| 14. | Rhoplex AC-34™ Acrylic Emulsion | 372 gm |
| 15. | Super Cobalt™ Drier | 1 gm |

Mixing continued for at least 1/2 hour at mixing speed. Other pigments, such as carbon black or red oxide of iron, may be added to this to replace part of the titanium dioxide ingredients in items 7 & 8 and provide a differing color balance. The above formulation was derived from Ernest Flick "Water-Based Paint Formulations" Noyes Publications, Parkridge, New Jersey.

### 9B. Oil Emulsion Paint

A satisfactory thixotropic paint was prepared as follows. Premix in a high speed stone mill:

| | | |
|---|---|---|
| 1. | Water | 205 ml |
| 2. | Victawet 35B™ Wetting agent | 4 ml |
| 3. | Potassium polyphosphate | 5 gm |

Adjust speed of the mill to disperse the following additives and pigments:

| | | |
|---|---|---|
| 4. | Ethylene glycol | 10 gm |
| 5. | Titanox RANC™ Rutile TiO₂ | 175 gm |
| 6. | Titanox A168L0™ Anatase TiO₂ | 50 gm |
| 7. | Asbestine 3X™ Talc | 50 gm |
| 8. | Zinc oxide AZO-ZZZ-33™ | 125 gm |
| 9. | Nuodex PMA-18 Mildewoide™ | 2 ml |
| 10. | Nopco NDW™ Defoamer | 2 ml |
| 11. | Victawet 35B™ Wetting agent | 16 gm |

The mill was then slowed to mixing speed and the following were added:

| | | |
|---|---|---|
| 12. | N. plumbaginifolia emulsion (2.5% w/w) | 130 ml |
| 13. | Emulsified linseed oil (60% solids) | 340 ml |
| 14. | Super Cobalt™ drier | 11 gm |

Milling was continued for approximately 1/2 hour.

This procedure resulted in a paint that tends to set to relatively stiff or "buttery" consistency upon standing, but thins down to relatively mobile liquid when mechanically agitated. This thixotropic character is such that the shearing action of the brush used to apply the paint to a surface is sufficient to render the paint adequately mobile. The paint leaving the brush remains fluid for a sufficient time to bring about good levelling (i.e., the brush marks disappear while the paint again sets to a stiff consistency before it has time to run appreciably on the surface painted).

This thixotropic property in paints is valuable in flat paints meant to be applied to interiors with a brush because it prevents the running of the paint and at the same time eliminates brush marks. Thixotropic paints possess a further advantage quite apart from their intended use for the reason above, in that the paint acquires a buttery or solid consistency upon standing in containers. Segregation or stratification of the paint during long periods of storage is thus prevented.

The above formulation and methods were derived from U.S. Patent No. 2,135,936, November 8, 1938, for "Use of gum arabic in paint" and from "Emulsion and Water Soluble Paints and Coatings."

### Example 10 - Ceramic Glazes

The suspension of the glaze ingredients in a glaze slip for several hours or even days has been achieved using N. plumbaginifolia gum product as an emulsifying and/or suspending agent. Further, the resulting glaze has superior clarity and stability.

A stable glaze slip was prepared as follows:

To prepare an emulsifying/stabilizing mixture, the following ware combined:

| | | |
|---|---|---|
| 1. | N. plumbaginifolia dry gum product (grown on BLM as carbon source) | 10 gm |
| 2. | Cold water | 500 cc |

### Example 11 - Clear Thixotropic Detergent or Cleaning Preparation

A satisfactory thixotropic cleaning detergent with superior grip and film forming properties was prepared as follows:

| | | |
|---|---|---|
| 1. | Water | 812 ml |
| 2. | N. plumbaginifolia (BLM carbon source) | 40 gm |
| 3. | Sodium chloride | 20 gm |

The gum product was added to the water in a high speed mixer running at a slow speed and was mixed for 15 minutes. The mixture was then left for 1-1/2 hours and the sodium chloride was added, mixing slowly for 3-5 minutes. Sodium ethylsulphate was then added while mixing continued:

| | | |
|---|---|---|
| 4. | Sodium ethylsulphate (C12-14 2E.0.) (100% basis) | 125 gm |
| 5. | Perfume | <.5 gm |
| 6. | Dye | <.5 gm |
| 7. | Preservative | <.5 gm |

The perfume, dye and preservative were then added, and mixing continued for another 10 minutes.

Because the foregoing formulation does not contain either ethyl alcohol or propylene glycol (which can be used in cleaning detergents), the possibility of precipitation of the gum due to a high alcohol concentration is averted.

The resulting product is a clear cleaning agent which tends to be relatively stiff and provides an adequate detergent which clings to the surfaces. This is a desirable characteristic particularly in the cleaning of vertical surfaces. It was found that the BLM carbon source enhanced the film forming properties of the detergent.

### Example 12 - Cosmetic Creams and Lotions

12.A. The concentrated gum from Nicotiana Batch 3-1000 (1.7% total solids) was found to have pleasant soft feeling on the skin and to dry without stickiness. When mixed with water, it makes a satisfying, i.e., moisturizing, skin treatment without any further additions. Another product was prepared by perfuming the biopolymer solution with 0.1% v/v rose oil.
12.B. A cosmetic lotion was prepared with the ingredients indicated below. The vegetable oil, perfuming oil and glycerol were added to the biopolymer solution while mixing with a high speed stirrer such as an Ultraturrax at a setting of about 6.

| | |
|---|---|
| Nicotiana gum #3-1000 | 2.4% (w/w in H₂0) |
| Orange oil | 1.0% (w/w) |
| Olive oil | 2.3% (w/w) |
| Glycerol | 5.3% (w/w) |

The Nicotiana gum was mixed in the water in a high speed stirrer such as an Ultraturrax at a setting of about 6. The olive oil, orange oil and glycerol were then added. The result was a soft gel with a pleasant fresh aroma which can be spread on the hands or face, leaving skin feeling fresh and soft.
12.C. A cosmetic lotion was prepared using the following:

| | |
|---|---|
| Nicotiana gum #3-1000 | 1.7% (w/w in H₂0) |
| Sunflower oil | 1.3% |
| Glycerol | 4.0% |

The ingredients were combined as in 11.B. The resulting product was soft enough to be used in a pump-action dispenser. A perfuming oil can be added if desired.
12.D. Other batches of gum from other cell lines were used to prepare products with different properties. For example, a cream was prepared from gum produced by Nicotiana cells growing in a medium containing Brewers Liquid Maltose ("BLM") 162 g/liter, as the source of sugar. The resulting gum produced a viscous solution and was used to prepare a lotion with the following formulation:

| | |
|---|---|
| Nicotiana gum | 2.3% (w/w in H₂0) |
| Peanut oil | 3.3% |
| Rose oil | 0.1% |
| Glycerol | 5.0% |

### Example 13 - Pharmaceutical Formulation

A lubricating jelly formulation in the pharmaceutical area was prepared using *P.communis* gum product (P1000).

| Material | Formulation |
|---|---|
| | % w/w |
| Distilled water | 77.8 |
| Propylene Glycol | 18.0 |
| P1000 | 4.0 |
| Imidazolidinyl Urea | 0.2 |

- Appearance:: Translucent, smooth, flowable gel.
- Skin Feel:: Slippery, silky feel (provides good lubrication)
- Viscosity (20°C):: 18,500 cps (spindle 4, speed 6, LVT Brookfield Viscometer)
- pH:: 4.5

This product has very good slip/lubricating properties and much of this characteristic could be attributable to the *P. communis* gum product in the formulation.

### Example 14 - Personal Care Products

The following skin moisturising lotion was prepared where *N. plumbaginifolia* gum product (NP1000) provided thickening and emulsion stabilising effects:

| Material | Formulation |
|---|---|
| | % w/w |
| Paraffin liquid | 6.0 |
| Bees Wax | 4.0 |
| White soft petroleum jelly | 10.0 |
| Liquid Paraffin | 10.0 |
| Span 20 | 2.0 |
| Tween 20 | 2.0 |
| Distilled water | 61.33 |
| Propylene Glycol | 3.0 |
| Triethanolamine (85%) | 0.5 |
| NP1000 | 1.0 |
| Citric acid | 0.05 |
| Kathon OG | 0.07 |
| Fragrance | 0.05 |

- Appearance:: White, smooth lotion.
- Viscosity (20°C):: 2,700 cps (spindle 3, speed 12 LVT Brookfield viscometer).
- pH:: 7.8.

This product provides an example of a common type of moisturising lotion. The NP1000 gum product provides some thickening and acts as an emulsion stabilizer by restricting the mobility of the internal phase.

### Example 15 - Household Products

In order to investigate the potential use off the plant gum products in the household product category, a toilet bowl cleaner was prepared using *P.communis* gum product (P1000).

| Material | Formulation |
|---|---|
| | % w/w |
| Distilled Water | 91.24 |
| Teric LA8N | 4.35 |
| P1000 | 2.0 |
| Citric acid | 1.90 |
| FD&C Blue#1 dye | 0.01 |
| Fragrance | 0.5 |

- Appearance:: Translucent, blue, slightly viscous liquid.
- pH:: 2.4
- Viscosity (20°C):: 60 cps (spindle 3, speed 30 LVT Brookfield iscometer).

The P1000 gum product provides some thickening to the product and helps the product "cling" to surfaces. This enables the product to be more substantive to the surfaces and therefore allows the surfactant more time to remove debris.

### Example 16 - Industrial/Automotive Formulations.

In order to establish the potential of plant gum products in the industrial category, an automotive cleaner/polish was prepared according to the following formulation, using *P*. *communis* gum product (P1000).

| Material | Formulation |
|---|---|
| | %w/w |
| Silicone oil 200/20 | 4.0 |
| Oleic acid | 2.0 |
| Mineral spirits | 20.0 |
| Distilled water | 66.75 |
| Triethanolamine (85%) | 0.2 |
| P1000 | 1.0 |
| Silica (Syloid 72) | 6.0 |
| Kathon ICP | 0.05 |

- Appearance:: Off-white, smooth lotion.
- Viscosity (20°C):: 1300 cps (spindle 3, speed 12 LVT Brookfield Viscometer).
- pH:: 7.2

In this role the P1000 gum product imparts some thickening properties and also contributes to the emulsion stability.

### Example 17 - Wool Sizing Agents

Two samples of plant gum products, *P.communis* gum product (GG) and *N. plumbaginifolia* gum product (FGG), were evaluated as sizing agents for all-wool yarns.
A. Solutions were prepared to give viscosities of around 200 cps at ambient temperature (this viscosity maximises application efficiency) as follows:
- GG: - a 1.5% (wt/wt) solution of GG was prepared, allowed to stand overnight, and pH adjusted to 6.5 (182 cps).
- FGG: - a 3% (wt/wt) solution prepared as above (205 cps).

B. Solution pumping rates and yarn speeds were adjusted to give a range of polysaccharide solids on the yarn from 2 to 6% (wt/wt). Samples of treated yarns were "desized" (washing, 1hr., 40°C, pH 8) to determine actual add-ons.

| **P/sacch.** | **Pump Ratio** | **Desize Value** |
|---|---|---|
| GG (1.5% solution) | 1 | 2.2 |
| | 2 | 2.9 |
| | 4 | 6.7 |
| FGG (3% solution) | 1 | 1.5 |
| | 2 | 3.0 |

C. Yarns were tested for increase in strength (load at break), extension at break, reduction of surface hairiness and resistance to surface abrasion.

| | **GG** | **FGG** |
|---|---|---|
| Yarn strength | + 5% | +2-5%. |
| Yarn extensibility | no loss | no loss |
| Surface hairiness (% solids level) | -25% (2) | -30% (1.5) |
| | -49% (3) | -35% (3) |
| | -67% (6.5) | |
| abrasion resistance | No improvement observed. | |

The observed reduction in surface hairiness, the small increase in yarn strength and the fact that the yarns did not become brittle all indicate potential for the materials as sizing agents for wool.

### Example 18 - Agricultural Formulations.

The *P.communis* gum product (p1000) or *N. plumbaginifolia* gum product (NP 1000) are used as thickening agents in the following typical agrochemical formulations, which are suspension concentrates of herbicides commonly used in broadacre agriculture:

| | **Material** | **Quantity** |
|---|---|---|
| (a) | Diuron Technical | 510.2 g |
| | Ethylene Glycol | 50.0 g |
| | Surfactants | 55.0 g |
| | Antifoam Agent | 1.3 g |
| | Thickening Agent | 3.0 g |
| | Other Components | 0.5 g |
| | Water | 390.0 g |
| (b) | Atrazine Technical | 510.0 g |
| | Ethylene Glycol | 50.0 g |
| | Dispersant/Wetting Agents | 34.3 g |
| | Surfactants | 9.5 g |
| | Thickening Agent | 3.4 g |
| | Antifoam Agent | 1.5 g |
| | Dye | 1.0 g |
| | Water | 401.0 g |

## Claims

1. An improved process for manufacturing an industrial, pharmaceutical or cosmetic product, said process excluding food manufacturing, which process comprises a step of including a viscosifying agent, thickening agent, gelling agent, emulsifying agent, suspending agent, stabilizing agent, encapsulating agent, flocculating agent, film-forming agent, sizing agent, adhesive agent, binding or coating agent, lubricating agent, water retention agent or coagulation agent or any combinations of such agents in the manufactured product, the improvement comprising using a cultured plant cell gum of a vascular plant as said agent.

2. The method of claim 1 wherein AGP concentration in said gum is at least about 4.0% (w/w) of said gum.

3. The method of claim 1 wherein said gum is employed as an emulsifying agent.

4. The method of claim 1 wherein cultured plant cell gum concentration in said culture broth is at least about 0.05% (w/v).

5. The method of claim 1 wherein the cultured plant cell gum is produced by Pyrus cells, Nicotiana cells, Phleum cells or Lolium cells.

6. An improved method of making a cultured Pyrus or Nicotiana plant cell gum comprising the steps of:
culturing a Pyrus or Nicotiana plant cell on a medium containing Brewers Liquid Maltose (BLM) as a carbon source in a concentration of between about 80 and 200g (wet weight) per liter of medium to produce a gum; and
recovering said gum.

7. An improved industrial, pharmaceutical or cosmetic manufacturing process, said process excluding food manufacturing, wherein the improvement comprises using a cultured plant cell gum produced by the method of claim 6.

8. A composition produced by the method of claim 1 selected from the group consisting of sheet paper, an adhesive, an oil and gas well fluid, an ink formulation, a lithography solution, a textile, a textile dyestuff, paint, ceramic glaze, cleaning detergent, a cosmetic lotion, a cosmetic cream, a lubricating jelly, a skin moisturizing lotion, a household cleaner, an automotive cleaner/polish, a wool sizing agent and an agrochemical formulation.

9. The composition of claim 8, wherein said gum is selected from the group consisting of Nicotiana, Pyrus, Phleum, and Lolium cultured plant cell gums.

10. The method of claim 1 selected from the group consisting of manufacturing processes for sheet paper, an adhesive, an oil and gas well fluid, an ink formulation, a lithography solution, a textile, a textile dyestuff, paint, ceramic glaze, cleaning detergent, a cosmetic lotion, a cosmetic cream, a lubricating jelly, a skin moisturizing lotion, a household cleaner, an automotive cleaner/polish, a wool sizing agent and an agrochemical formulation.

11. The oil and gas well fluid of claim 8, selected from the group consisting of a drilling fluid, a cementing fluid, a completion fluid, and an enhanced oil recovery injection fluid.

12. An oil and gas well fluid comprising a cultured plant cell gum of a vascular plant.

13. The oil and gas well fluid of claim 12, wherein said gum is selected from the group consisting of Nicotiana, Pyrus, Phleum, and Lolium cultured plant cell gums.

14. The oil and gas well fluid of claim 12, selected from the group consisting of a drilling fluid, a cementing fluid, a completion fluid, and an enhanced oil recovery injection fluid.

15. In a method of making an oil and gas well fluid, said method employing a thickener, or an emulsifying, suspending, lubricating or fluid loss reduction agent, an improvement comprising using a cultured plant cell gum of a vascular plant as a thickener, or an emulsifying, suspending, lubricating or fluid loss reduction agent, or any combination thereof.

16. The lithography solution of claim 8, selected from the group consisting of a fountain solution, a sensitizing solution and a protecting solution.

17. The paint of claim 8 selected from the group consisting of oil emulsion paint and acrylic resin paint.

18. In a method of ceramic forming by extrusion, said method employing a plasticizer, an improvement comprising using a cultured plant cell gum of a vascular plant as a plasticizer.

19. In a method of ceramics slip casting, said method employing a suspending agent, an improvement comprising using a cultured plant cell gum of a vascular plant as a suspending agent.

20. A plant gum product comprising glycoprotein and complex carbohydrate, produced by a method which comprises the steps of:
(a) culturing gum-secreting plant cells derived from tissues of vascular plants in suspension culture in the presence of a culture medium; and
(b) recovering the gum product secreted by the cells from the culture medium,
wherein the plant cells are derived from *Nicotiana*, *Phleum*, *Lolium* or Timothy grass.

21. A plant gum product according to claim 20, derived from Nicotiana cells, Phleum cells or Lolium cells

22. A plant gum product according to claim 21, derived from *Nicotiana plumbaginifolia*, *Nicotiana alata* cells, *Phleum pratense* cells or *Lolium multiflorum* cells.

23. An improved industrial, pharmaceutical or cosmetic product, excluding a food product, characterized in that it contains a plant gum product secreted by suspension-cultured gum-secreting plant cells derived from the tissues of vascular plants.

24. An improved product according to claim 23, selected from the group consisting of sheet paper, an adhesive, an oil and gas well fluid, an ink formulation, a lithography solution, a textile, a textile dyestuff, paint, ceramic glaze, cleaning detergent, a cosmetic lotion, a cosmetic cream, a lubricating jelly, a kin moisturizing lotion, a household cleaner, an automotive cleaner/polish, a wool sizing agent and an agrochemical formulation.

25. An improved product according to claim 23 or claim 24, characterized in that it contains a plant gum product according to claim 20.

26. The improved process of claim 7 wherein said cultured plant cell gum is employed as a film-forming agent.

27. In an industrial, pharmaceutical or cosmetic manufacturing process in which a plant exudate or plant extract gum is employed as a thickening, emulsifying, suspending, waterproofing, gelling, protective colloid, stabilizing or coating agent, the improvement wherein said plant exudate or extract gum is replaced with a cultured plant cell gum of a vascular plant.

28. The process of claim 27 in which gum arabic is used as a viscosifying, emulsifying or gelling agent, the improvement wherein a cultured plant cell gum of a Pyrus plant is substituted for gum arabic.

29. The process of claim 27 in which guar gum or hydroxymethyl-cellulose is used as a viscosifying agent, the improvement wherein a cultured plant cell gum of a Phleum plant is substituted for guar gum or hydroxymethyl-cellulose.

30. The process of claim 27 wherein gum arabic is used as a viscosifying and coating agent, the improvement wherein a cultured plant cell gum of a Nicotiana plant is substituted for said gum arabic.

## Patentansprüche

1. Verbessertes Verfahren zur Herstellung eines industriellen, pharmazeutischen oder kosmetischen Erzeugnisses, wobei das Verfahren die Lebensmittelherstellung ausschließt und eine Stufe umfaßt, in welcher ein Viskositätsmittel, Verdickungsmittel, Geliermittel, Emulgator, Suspendiermittel, Stabilisierungsmittel, Verkapselungsmittel, Flockungsmittel, filmbildendes Mittel, Schlichtemittel, Klebstoff, Binde- oder Beschichtungsmittel, Gleitmittel, Wasserrückhaltemittel oder Gerinnungsmittel oder beliebige Kombinationen solcher Mittel in das hergestellte Erzeugnis eingebaut wird/werden, wobei die Verbesserung die Verwendung eines kultivierten Pflanzenzellgummis einer Leitgewebe enthaltenden Pflanze als dieses Mittel umfaßt.

2. Verfahren nach Anspruch 1, worin die Arabinogalactanprotein-Konzentration in dem Gummi mindestens etwa 4,0 Gew.-% des Gummis beträgt.

3. Verfahren nach Anspruch 1, worin das Gummi als Emulgator verwendet wird.

4. Verfahren nach Anspruch 1, worin die Konzentration des kultivierten Pflanzenzellgummis in der Kulturbrühe mindestens etwa 0,05 % (Gew./Vol.) beträgt.

5. Verfahren nach Anspruch 1, worin das kultivierte Pflanzenzellgummi durch Pyrus-, Nicotiana-, Phleum- oder Lolium-Zellen hergestellt wird.

6. Verbessertes Verfahren zur Herstellung eines kultivierten Pyrus- oder Nicotiana-Pflanzenzellgummis, welches die Stufen
- Kultivierung von Pyrus- oder Nicotiana-Pflanzenzellen in einem Medium, das als Kohlenstoffquelle flüssige Braumaltose (BLM) in einer Konzentration von zwischen etwa 80 und 200 g (Naßgewicht) pro Liter Medium enthält, um ein Gummi zu bilden, und
- Gewinnung des Gummis
umfaßt.

7. Verbessertes industrielles, pharmazeutisches oder kosmetisches Herstellungsverfahren, wobei die Lebensmittelherstellung ausgeschlossen ist und worin die Verbesserung die Verwendung eines kultivierten Pflanzenzellgummis umfaßt, das durch das Verfahren des Anspruchs 6 hergestellt ist.

8. Zusammensetzung, welche durch das Verfahren des Anspruchs 1 hergestellt und aus der Gruppe ausgewählt ist, die aus einem Blatt Papier, einem Klebstoff, einem Erdöl- und Erdgasbohrlochfluid, einer Druckfarbenformulierung, einer Lithographielösung, einem Gewebe, einem Textilfarbstoff, einem Anstrichstoff, einer Keramikglasur, einem Reinigungsmittel, einer Kosmetiklotion, einer Kosmetikcreme, einem Gleitgel, einer Hautfeuchthaltelotion, einem Haushaltsreiniger, einem Fahrzeugreiniger und -polish, einem Wollschlichtemittel und einer agrochemischen Formulierung besteht.

9. Zusammensetzung nach Anspruch 8, worin das Gummi aus der Gruppe ausgewählt ist, die aus kultivierten Nicotiana-, Pyrus-, Phleum- und Lolium-Pflanzenzellgummen besteht.

10. Verfahren nach Anspruch 1, welches aus der Gruppe ausgewählt ist, die aus den Herstellungsverfahren für Papierblätter, Klebstoffe, Erdöl- und Erdgasbohrlochfluide, Druckfarbenformulierungen, Lithographielösungen, Gewebe, Textilfarbstoffe, Anstrichstoffe, Keramikglasuren, Reinigungsmittel, Kosmetiklotionen, Kosmetikcremes, Gleitgele, Hautfeuchthaltelotionen, Haushaltreiniger, Fahrzeugreiniger und -polishs, Wollschlichtemittel und agrochemische Formulierungen besteht.

11. Erdöl- und Erdgasbohrlochfluid nach Anspruch 8, welches aus der Gruppe ausgewählt ist, die aus einem Bohrspülmittel , einem Zementierfluid, einem Abschlußfluid und einem Einpreßfluid der tertiären Erdölförderung besteht.

12. Erdöl- und Erdgasbohrlochfluid, das ein kultiviertes Pflanzenzellgummi einer Leitgewebe enthaltenden Pflanze umfaßt.

13. Erdöl- und Erdgasbohrlochfluid nach Anspruch 12, worin das Gummi aus der Gruppe ausgewählt ist, die aus kultivierten Nicotiana-, Pyrus-, Phleum- und Lolium-Pflanzenzellgummen besteht.

14. Erdöl- und Erdgasbohrlochfluid nach Anspruch 12, das aus der Gruppe ausgewählt ist, die aus einem Bohrspülmittel, einem Zementierfluid, einem Abschlußfluid und einem Einpreßfluid der tertiären Erdölförderung besteht.

15. In einem Verfahren zur Herstellung eines Erdöl- und Erdgasbohrlochfluids, in welchem ein Verdickungsmittel oder ein Emulgator, Suspendiermittel, Schmiermittel oder Fluid-loss Reducer eingesetzt wird, umfaßt eine Verbesserung die Verwendung eines kultivierten Pflanzenzellgummis einer Leitgewebe enthaltenden Pflanze als Verdickungsmittel oder Emulgator, Suspendiermittel, Schmiermittel oder Fluid-loss Reducer.

16. Lithographielösung nach Anspruch 8, welche aus der Gruppe ausgewählt ist, die aus einer Sprühlösung, Sensibilisierlösung und einer Schutzlösung besteht.

17. Anstrichstoff nach Anspruch 8, welcher aus der Gruppe ausgewählt ist, die aus Ölemulsionsanstrichstoffen und Acrylharzanstrichstoffen besteht.

18. In einem Verfahren zur Formgebung von Keramik durch Strangpressen, in welchem ein Plastifizierungsmittel eingesetzt wird, umfaßt eine Verbesserung die Verwendung eines kultivierten Pflanzenzellgummis einer Leitgewebe enthaltenden Pflanze als Plastifizierungsmittel.

19. In einem Verfahren zum Gießen von Keramikschlicker, in welchem ein Suspendiermittel eingesetzt wird, umfaßt eine Verbesserung die Verwendung eines kultivierten Pflanzenzellgummis einer Leitgewebe enthaltenden Pflanze als Suspendiermittel.

20. Pflanzengummiprodukt, welches Glycoprotein und komplexes Kohlenhydrat enthält und durch ein Verfahren hergestellt wird, das die Stufen
(a) Kultivierung Gummi-absondernder Pflanzenzellen, die von Geweben Leitgewebe enthaltender Pflanzen abgeleitet sind, in einer Suspensionskultur in Anwesenheit eines Kulturmediums und
(b) Gewinnung des von den Zellen abgesonderten Gummiprodukts aus dem Kulturmedium
umfaßt, wobei die Pflanzenzellen von *Nicotiana*, *Phleum*, *Lolium* oder *Wiesenlieschgras* abgeleitet sind.

21. Pflanzengummiprodukt nach Anspruch 20, das von *Nicotiana*-, *Phleum*- oder *Lolium*-Zellen abgeleitet ist.

22. Pflanzengummiprodukt nach Anspruch 21, das von Zellen von *Nicotiana plumbaginifolia*, *Nicotiana alata*, *Phleum pratense* oder *Lolium multiflorum* abgeleit ist.

23. Verbessertes industrielles, pharmazeutisches oder kosmetisches Erzeugnis, ausschließlich eines Lebensmittelerzeugnisses**, dadurch gekennzeichnet, daß** es ein Pflanzengummiprodukt enthält, das von in Suspension kultivierten, Gummi-absondernden Pflanzenzellen abgesondert ist, die von Geweben Leitgewebe enthaltender Pflanzen abgeleitet worden sind.

24. Verbessertes Erzeugnis nach Anspruch 23, welches aus der Gruppe ausgewählt ist, die aus einem Blatt Papier, einem Klebstoff, einem Erdöl- und Erdgasbohrlochfluid, einer Druckfarbenformulierung, einer Lithographielösung, einem Gewebe, einem Textilfarbstoff, einem Anstrichstoff, einer Keramikglasur, einem Reinigungsmittel, einer Kosmetiklotion, einer Kosmetikcreme, einem Gleitgel, einer Hautfeuchthaltelotion, einem Haushaltsreiniger, einem Fahrzeugreiniger und -polish, einem Wollschlichtemittel und einer agrochemischen Formulierung besteht.

25. Verbessertes Erzeugnis nach Anspruch 23 oder 24, **dadurch gekennzeichnet, daß** es ein Pflanzengummiprodukt gemäß Anspruch 20 enthält.

26. Verbessertes Verfahren nach Anspruch 7, Worin das kultivierte Pflanzenzellgummi als filmbildendes Mittel eingesetzt wird.

27. In einem industriellen, pharmazeutischen oder kosmetischen Herstellungsverfahren, in welchem ein Pflanzenexsudat- oder Pflanzenextraktgummi als Verdickungsmittel, Emulgator, Suspendiermittel, wasserabweisendes Mittel, Geliermittel, Schutzkolloid, Stabilisierungs- oder Beschichtungsmittel verwendet wird, besteht die Verbesserung darin, daß das Pflanzenexsudat- oder Pflanzenextraktgummi durch ein kultiviertes Pflanzenzellgummi einer Leitgewebe enthaltenden Pflanze ersetzt wird.

28. Verfahren nach Anspruch 27, worin Gummi arabicum als Viskositäts-, Emulgier- oder Geliermittel verwendet wird, wobei die Verbesserung darin besteht, daß das Gummi arabicum durch ein kultiviertes Pflanzenzellgummi einer Pyrus-Pflanze substituiert wird.

29. Verfahren nach Anspruch 27, worin Guar-Gummi oder Hydroxymethylcellulose als Viskositätsmittel verwendet wird, wobei die Verbesserung darin besteht, daß das Guar-Gummi oder die Hydroxymethylcellulose durch ein kultiviertes Pflanzenzellgummi einer Phleum-Pflanze substituiert ist.

30. Verfahren nach Anspruch 27, worin Gummi arabicum als Viskositäts- und Beschichtungsmittel verwendet wird, wobei die Verbesserung darin besteht, daß das Gummi arabicum durch ein kultiviertes Pflanzenzellgummi einer Nicotiana-Pflanze substituiert wird.

## Revendications

1. Procédé amélioré destiné à la préparation d'un produit industriel, pharmaceutique ou cosmétique, ledit procédé excluant la préparation de produits alimentaires, lequel procédé comprend une étape consistant à inclure un agent augmentant la viscosité, un agent épaississant, un agent gélifiant, un agent émulsifiant, un agent de suspension, un agent stabilisant, un agent d'encapsulation, un agent floculant, un agent filmogène, un agent d'encollage, un agent adhésif, un agent liant ou un agent de couchage, un agent lubrifiant, un agent hydratant ou un agent de coagulation ou toute combinaison de tels agents dans le produit fabriqué, l'amélioration comprenant une gomme de plante vasculaire produite par culture de cellules végétales comme dit agent.

2. Méthode selon la revendication 1 dans laquelle la concentration d'AGP dans ladite gomme est d'au moins environ 4,0 % (poids/poids) de ladite gomme.

3. Méthode selon la revendication 1 dans laquelle ladite gomme est utilisée comme agent émulsifiant.

4. Méthode selon la revendication 1 dans laquelle la concentration de la gomme produite par culture de cellules végétales dans ledit bouillon de culture est d'au moins environ 0,05 % (poids/volume).

5. Méthode selon la revendication 1 dans laquelle la gomme produite par culture de cellules végétales est produite par des cellules de Pyrus, des cellules de Nicotiana, des cellules de Phleum ou des cellules de Lolium.

6. Méthode améliorée de préparation d'une gomme produite par culture de cellules végétales de Pyrus ou de Nicotiana comprenant les étapes consistant à :
cultiver des cellule végétales de Pyrus ou Nicotiana dans un milieu contenant du maltose liquide de brasserie (Brewers Liquid Maltose, BLM) comme source de carbone à une concentration comprise entre 80 et 200 g (poids frais) par litre de milieu pour produire une gomme ; et
récupérer ladite gomme.

7. Procédé de fabrication industriel, pharmaceutique ou cosmétique amélioré, ledit procédé excluant la préparation de produits alimentaires, dans lequel l'amélioration comprend l'utilisation d'une gomme produite par culture de cellules végétales selon la méthode de la revendication 6.

8. Composition produite par la méthode selon la revendication 1 choisie dans le groupe constitué d'une feuille de papier, un adhésif, un fluide d'extraction pétrolière et gazière, une formulation d'encre, une solution lithographique, un textile, un colorant de textile, une peinture, une glaçure pour céramique, un détergent de nettoyage, une lotion cosmétique, une crème cosmétique, un gel lubrifiant, un humectant pour la peau, un détergent domestique, un détergent/lustrant pour voiture, un agent d'encollage de la laine et une formulation agrochimique.

9. Composition selon la revendication 8, dans laquelle ladite gomme est choisie dans le groupe constitué de gommes produites par culture de cellules végétales de Nicotiana, Pyrus, Phleum et Lolium.

10. Méthode selon la revendication 1 choisie dans le groupe constitué de procédés de fabrication d'une feuille de papier, d'un adhésif, d'un fluide d'extraction pétrolière et gazière, d'une formulation d'encre, d'une solution lithographique, d'un textile, d'un colorant de textile, d'une peinture, d'une glaçure pour céramique, d'un détergent de nettoyage, d'une lotion cosmétique, d'une crème cosmétique, d'un gel lubrifiant, d'un humectant pour la peau, d'un détergent domestique, d'un détergent/lustrant pour automobile, d'un agent d'encollage de la laine et d'une formulation agrochimique.

11. Fluide d'extraction pétrolière et gazière selon la revendication 8, choisi dans le groupe constitué d'un fluide de forage, un fluide de cimentation, un fluide de complétion, et un fluide d'injection pour récupération assistée des hydrocarbures.

12. Fluide d'extraction pétrolière et gazière comprenant une gomme produite par culture de cellules végétales d'une plante vasculaire.

13. Fluide d'extraction pétrolière et gazière selon la revendication 12 dans lequel ladite gomme est choisie dans le groupe constitué de gommes produites par culture de cellules végétales de Nicotiana, Pyrus, Phleum et Lolium.

14. Fluide d'extraction pétrolière ou gazière selon la revendication 12 choisi dans le groupe constitué d'un fluide de forage, un fluide de cimentation, un fluide de complétion, et un fluide d'injection pour récupération assistée des hydrocarbures.

15. Amélioration dans une méthode de préparation d'un fluide d'extraction pétrolière et gazière, ladite méthode utilisant un épaississant, ou un émulsifiant, un agent de suspension, un lubrifiant ou un agent réducteur de filtrat, comprenant l'utilisation d'une gomme de plante vasculaire produite par culture de cellules végétales comme épaississant ou émulsifiant, agent de suspension, lubrifiant ou réducteur de filtrat, ou toute combinaison de ceux-ci.

16. Solution lithographique selon la revendication 8 choisie dans le groupe constitué d'une solution de mouillage, une solution de couchage et une solution de protection.

17. Peinture selon la revendication 8 choisie dans le groupe constitué d'une peinture d'émulsion dans l'huile ou d'une peinture à base de résine acrylique.

18. Amélioration dans une méthode de production de céramique par extrusion, ladite méthode utilisant un plastifiant, comprenant l'utilisation d'une gomme de plante vasculaire produite par culture de cellules végétales comme plastifiant.

19. Amélioration dans une méthode de barbotine de coulage pour céramique, ladite méthode utilisant un agent de suspension, comprenant l'utilisation d'une gomme de plante vasculaire produite par culture de cellules végétales comme agent de suspension.

20. Produit de gomme végétale comprenant une glycoprotéine et un hydrate de carbone complexe produit par une méthode qui comprend les étapes consistant à :
(a) cultiver des cellules végétales sécrétant de la gomme provenant de tissus de plantes vasculaires, par culture en suspension dans un milieu de culture ; et
(b) récupérer le produit de la gomme sécrété par les cellules à partir du milieu de culture, dans lequel les cellules végétales proviennent de *Nicotiana*, *Phleum*, *Lolium* ou fléole des prés.

21. Produit de gomme végétale selon la revendication 20, provenant de cellules de *Nicotiana*, de cellules de *Phleum* ou de cellules de *Lolium*.

22. Produit de gomme végétale selon la revendication 21, provenant de cellules de *Nicotiana plumbaginifolia*, *Nicotiana alata*, *Phleum pratense* ou *Lolium multiflorum*.

23. Produit industriel, pharmaceutique ou cosmétique amélioré, excluant un produit alimentaire, caractérisé en ce qu'il contient un produit gomme végétale sécrété par des cellules végétales cultivées en suspension sécrétant de la gomme et provenant de tissus de plantes vasculaires.

24. Produit amélioré selon la revendication 23 choisi dans le groupe constitué d'une feuille de papier, un adhésif, un fluide d'extraction pétrolière et gazière, une formulation d'encre, une solution lithographique, un textile, un colorant de textile, une peinture, une glaçure pour céramique, un détergent de nettoyage, une lotion cosmétique, une crème cosmétique, un gel lubrifiant, un humectant pour la peau, un détergent domestique, un détergent/lustrant pour automobile, un agent d'encollage de la laine et une formulation agrochimique.

25. Produit amélioré selon la revendication 23 ou la revendication 24, caractérisé en ce qu'il contient un produit gomme végétale selon la revendication 20.

26. Procédé amélioré selon la revendication 7 dans lequel ladite gomme produite par culture de cellules végétales est utilisée comme agent filmogène.

27. Amélioration dans un procédé de fabrication industriel, pharmaceutique ou cosmétique dans lequel un exsudat de plante ou une gomme extraite de plante est utilisé comme épaississant, émulsifiant, agent de suspension, imperméabilisant, gélifiant, colloïde protecteur, stabilisant ou agent de couchage, dans laquelle ledit exsudat de plante ou gomme extraite est remplacé par une gomme de plante vasculaire produite par culture de cellules végétales.

28. Procédé selon la revendication 27 dans lequel la gomme arabique est utilisée comme agent augmentation de la viscosité, comme agent émulsifiant ou gélifiant, l'amélioration consistant à remplacer la gomme arabique par une gomme produite par culture de cellules végétales de Pyrus.

29. Procédé selon la revendication 27 dans lequel la gomme guar ou hydroxyméthylcellulose est utilisée comme agent d'augmentation de la viscosité, l'amélioration consistant à remplacer la gomme guar ou l'hydroxyméthylcellulose par une gomme produite par culture de cellules végétales de Phleum.

30. Procédé selon la revendication 27 dans lequel la gomme arabique est utilisée comme agent d'augmentation de la viscosité ou de couchage, l'amélioration consistant à remplacer ladite gomme arabique par une gomme produite par culture de cellules végétales de Nicotiana.
